# EUROPEAN PATENT APPLICATION

(11) **EP 3 424 522 A1**
(43) Date of publication of application: **09.01.2019**
(21) Application number: 18173217.3
(22) Date of filing: 15.03.2011
(51) Int. Cl.: A61K 39/00

(54) **SYSTEM AND METHOD OF PREPARING AND STORING ACTIVATED MATURE DENDRITIC CELLS**

(30) Priority: 15.03.2010 US 31398410 P
(62) Divisional of application: 11756841.0
(71) Applicant: The Trustees of The University of Pennsylvania, Philadelphia PA 19104-6283 (US)
(72) Inventor: Czerniecki, Brian J., Tampa, FL 33602 (US); Koldovsky, Ursula, deceased (US); Xu, Shuwen, Garnet Valley, PA 19060 (US); Koski, Gary K., Akron, OH 44333 (US)
(74) Representative: Grünecker Patent- und Rechtsanwälte PartG mbB

(57) **Abstract**

The present invention provides compositions and methods for generating and cryopreserving dendritic cells with superior functionality in producing stronger signals to T cells, resulting in a more potent DC-based anti-tumor vaccine. The present invention includes mature, antigen loaded DCs activated by Toll-like receptor agonists that induce clinically effective immune responses, preferably when used earlier in the disease process. The DCs of the present invention produce desirable levels of cytokines and chemokines, and further have the capacity to induce apoptosis of tumor cells. The cells can be cryopreserved and thawed for later use, thereby reducing the need for repeated pheresis and elutriation processes during vaccine production. These methods can also be utilized to directly target molecules involved in carcinogenetic signaling pathways and cancer stem cells.

## Description

### BACKGROUND OF THE INVENTION

Cancer research has seen significant advances that have led to the steady reduction in mortality rates for many types of malignancies. This reduction in mortality rate has been influenced by improvements in early detection, advanced surgical techniques and the employment of novel therapeutic interventions. Given the success in decreasing cancer-related mortality rates, there has been a shift in research to focus on novel targeted therapies against cancer, of which the development of vaccines has been in the forefront. Vaccines have been highly effective in reducing mortality from pathogens due to their ability to activate the immune system and render immunity to foreign antigens. Not only does effective vaccination facilitate reduction in mortality, but vaccines also induce long-term immunity that protects against recurrent infection.

It is this ability of vaccines to develop immunity that initially garnered interest in developing cancer vaccines. Clearly, cancer vaccines of all forms have demonstrated at least some success in inducing immune responses against the tumor antigens selected for targets. While cancer vaccines have been generally disappointing when utilized as a stand-alone therapy, particularly in late disease settings (Terrando et al., 2007, Vaccine 25,4-16; Burgdorf et al., 2008, Oncol. Rep. 20(6), 1305-1311), recent studies suggest that dendritic cellular (DC) vaccines may impact patient survival.

Currently, methods used for immunotherapy typically incorporate *ex vivo* stimulated antigen presenting cells (APC), where DC precursors are cultured immediately after collection from the patient, and then antigen loaded DCs are infused into the patient as soon as they are harvested. Such methods create time constraints that not only limit the potential therapeutic uses for DC, but also necessitate pheresis products from the patient at multiple time points during the course of immunotherapy.

In addition to antigen presentation, it is believed that DCs, when matured, can further affect immune response by producing a variety of signal molecules, in the way of cytokines and chemokines. However, previous methods of DC immunotherapy did not utilize mature DCs. Further, previous work on the maturation of DCs did not fully optimize the maturation process to take advantage of DC signal production.

Because antigen loaded, mature DCs have already processed the antigen and have the ability to present antigen to the immune cells, these cells, when activated can not only quickly generate antigen specific immune responses, but they can also produce signal to further effect immune response. There is no doubt that effective cryopreservation of antigen loaded, mature DCs could provide a method of quickly generating functionally potent cells for immunotherapy. The cryopreservation of such cells has not yet been demonstrated effectively. Thus, there is a long felt need in the art for efficient and directed means of cyropreserving activated DCs in a manner that retains effective antigen presentation and signal secretion profiles of freshly generated DCs. The present invention satisfies this need.

### BRIEF SUMMARY OF THE INVENTION

The invention includes a method of generating antigen loaded, activated dendritic cells (DC) for use in immunotherapy, the method comprises: loading at least one antigen into a DC; activating the DC with at least one TLR agonist; cryopreserving the DC; and thawing the DC; wherein the DC produces an effective amount of at least one cytokine to generate a T cell response.

In one embodiment, the antigen is a tumor antigen. In another embodiment, the antigen is a microbial antigen. In yet another embodiment, the TLR agonist is LPS. In yet another embodiment, the cryopreserving comprises freezing the DC at a temperature of about -70°C or lower. In yet another embodiment, the recovery and viability of the DC after thawing is greater than or equal to about 70%. In yet another embodiment, the recovery and viability of the DC after thawing is greater than or equal to about 80%. In yet another embodiment, the DC are cryopreserved for at least about one week. In yet another embodiment, the cytokine is IL12. In yet another embodiment, the DC exhibits a killer function whereby the DC are capable of lysing targeted cancer cells.

The invention further includes a method of eliciting an immune response in a mammal, the method comprises administering a previously cryopreserved composition comprising an antigen loaded, activated DC into the mammal in need thereof, wherein the DC is antigen loaded and activated prior to being cryopreserved.

In one embodiment, the antigen is a tumor antigen. In another embodiment, the antigen is a microbial antigen. In yet another embodiment, the TLR agonist is LPS. In yet another embodiment, the cryopreserving comprises freezing the DC at a temperature of about -70°C or lower. In yet another embodiment, the recovery and viability of the DC after thawing is greater than or equal to about 70%. In yet another embodiment, the recovery and viability of the DC after thawing is greater than or equal to about 80%. In yet another embodiment, the DC are cryopreserved for at least about one week. In yet another embodiment, the cytokine is IL12. In yet another embodiment, the DC exhibits a killer function whereby the DC are capable of lysing targeted cancer cells.

The invention further includes a preservable composition for eliciting an immune response in a mammal, the composition comprises: a DC loaded with at least one antigen; wherein the DC has been activated by exposure to at least one TLR agonist; and wherein the DC produces an effective amount of at least one cytokine to generate a T cell response, irrespective of whether or not the composition has been cryopreserved.

In one embodiment, the antigen is a tumor antigen. In another embodiment, the antigen is a microbial antigen. In yet another embodiment, the TLR agonist is LPS. In yet another embodiment, the composition has been cryopreserved at a temperature of about -70°C or lower. In yet another embodiment, the recovery and viability of the DC after thawing is greater than or equal to about 70%. In yet another embodiment, the recovery and viability of the DC after thawing is greater than or equal to about 80%. In yet another embodiment, the composition is cryopreserved for at least about one week. In yet another embodiment, the cytokine is IL12. In yet another embodiment, the DC exhibits a killer function whereby the DC are capable of lysing targeted cancer cells.

### BRIEF DESCRIPTION OF THE DRAWINGS

For the purpose of illustrating the invention, there are depicted in the drawings certain embodiments of the invention. However, the invention is not limited to the precise arrangements and instrumentalities of the embodiments depicted in the drawings.

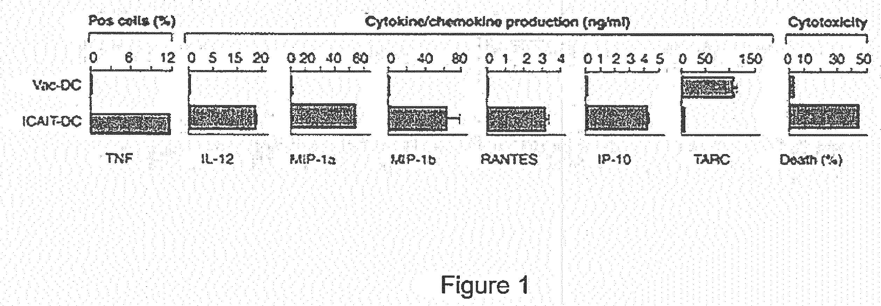

Figure 1 compares traditional DC (vac-DC) and ICAIT-DC for the production of a panel of cytokines and chemokines, as well as a killer function whereby these cells are capable of lysing breast cancer lines.

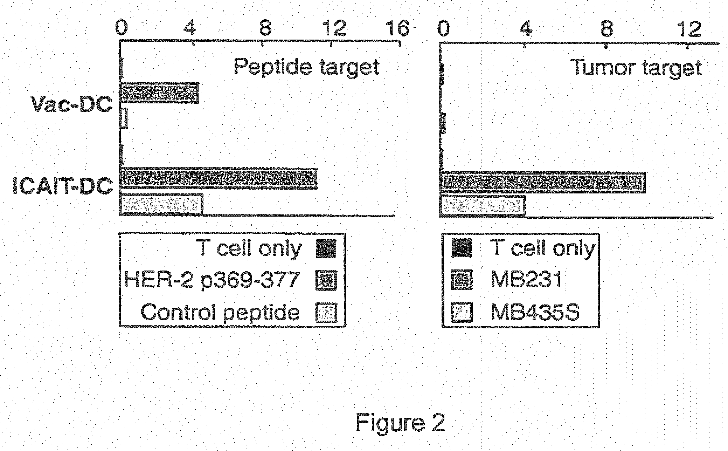

Figure 2 depicts both traditionally matured DC and ICAIT-DC successfully sensitizing T cells against tumor antigen, whereas only ICAIT-DC conditions T cells for actual recognition of HER-2-expressing tumors.

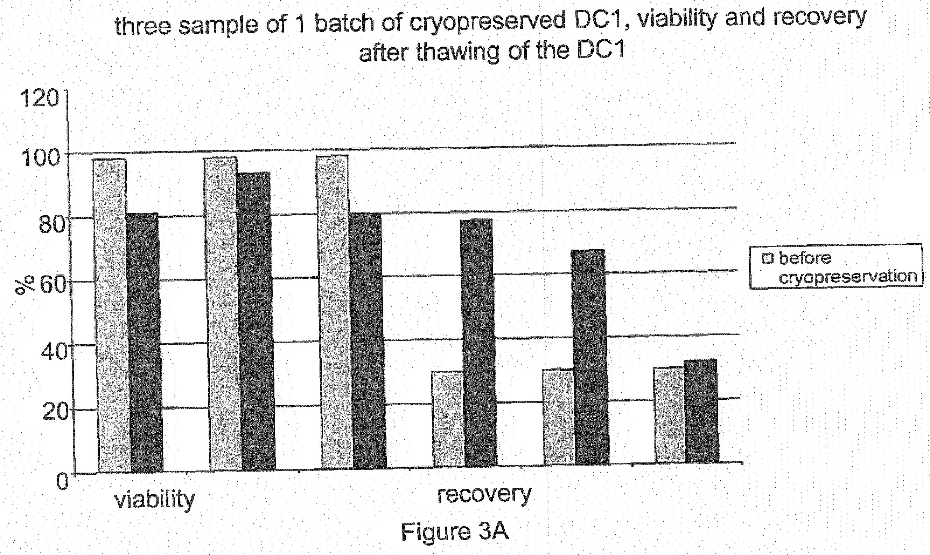

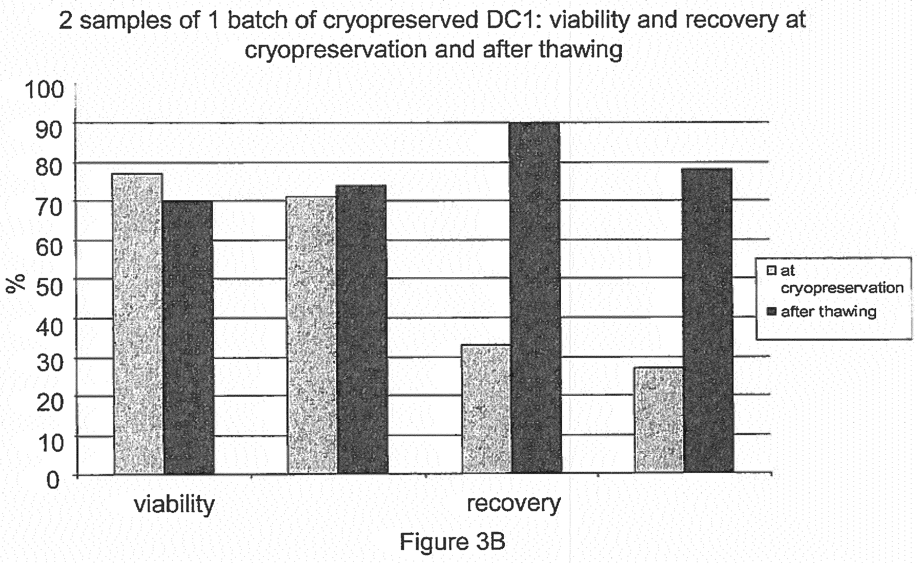

Figure 3, comprising Figures 3A and 3B, depicts comparable viability and recovery rates of fresh verses cryopreserved and thawed ICAIT-DCs. Using trypan blue exclusion, the viability and recovery rate of cryopreserved ICAIT DCs were determined before cryopreservation and immediately after thawing and washing (by centrifugation) of the cryopreserved cells. Figure 3A depicts three single cases from a two batch set, while Figure 3B depicts two single cases from a two batch set. The viability is comparable between freshly prepared and cryopreserved DC1s. The recovery rate of cryopreserved DC1s is generally between 80-90%, and much better than from freshly prepared DC1, due to the loss of cells by harvesting of freshly prepared DC1s.

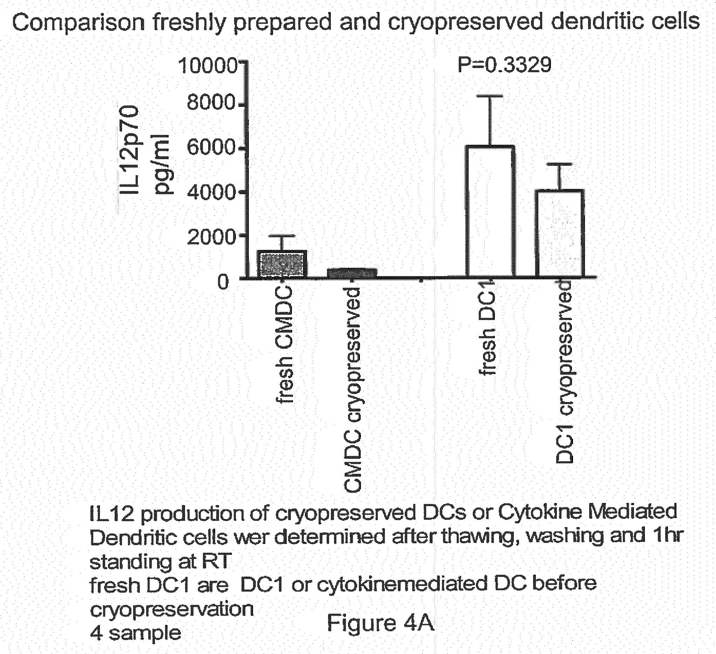

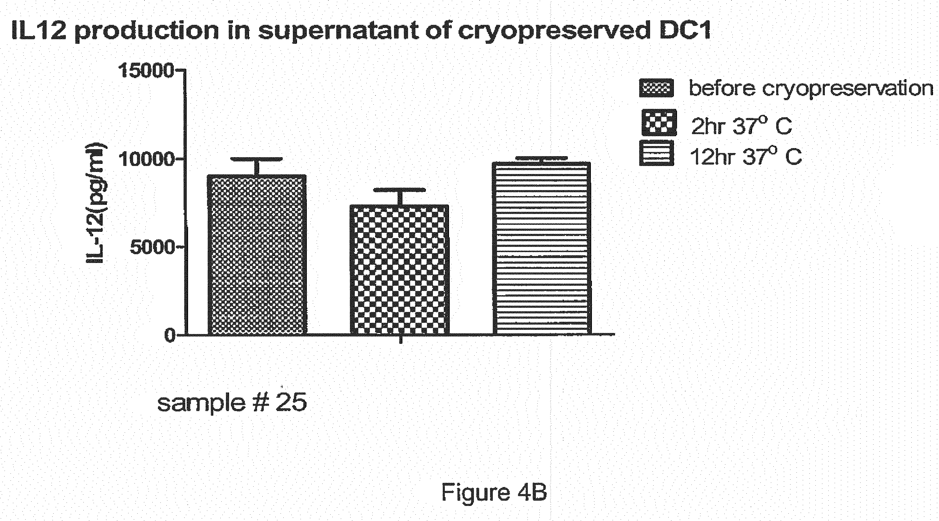

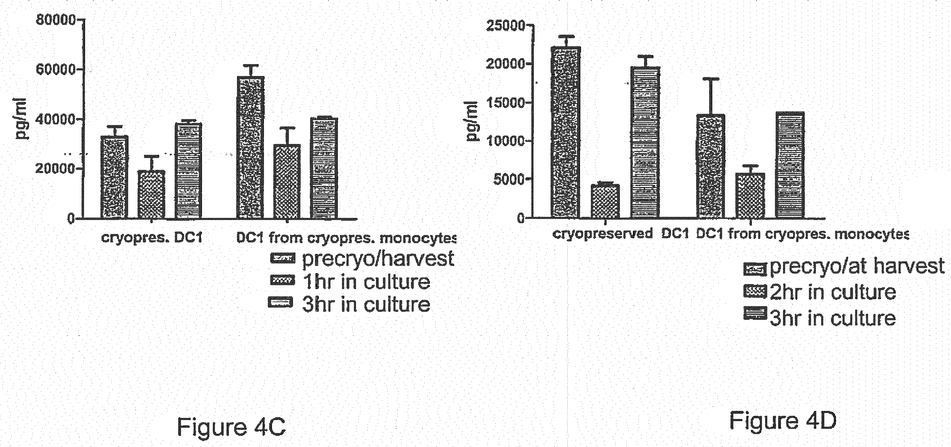

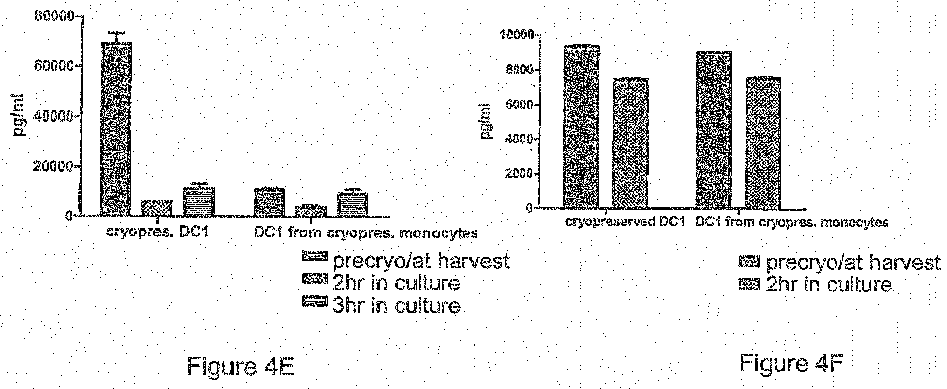

Figure 4, comprising Figures 4A-4F, depicts superior IL12 production levels of DC1s after thawing from cryopreservation. The continuing production of signal 3 (IL12) was measured by an ELISA Assay. Figure 4A depicts IL12 production in both fresh and cryopreserved cytokine mediated DCs (CMDCs) is far less than the IL12 production observed in both fresh and cryopreserved DC1s. Figure 4B depicts IL12 production in DC1s before cryopreservation, after 2 hrs after thawing and at 12 hrs after thawing. Figures 4C-4F depict IL12 production by cryopreserved DC1s in comparison to DC1s prepared from cryopreserved monocytes.

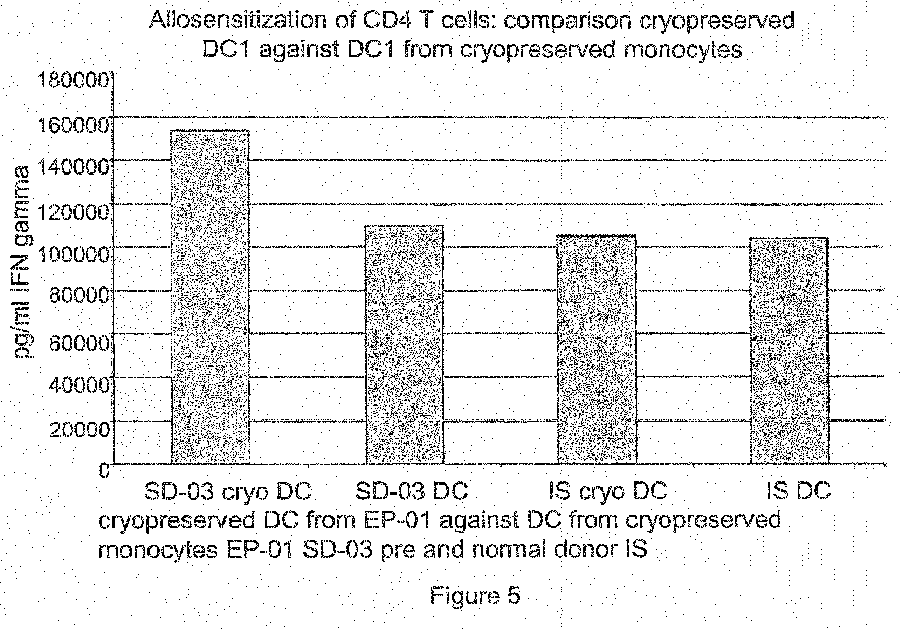

Figure 5 depicts IFNγ levels measured in cryopreserved DC1s in comparison with DC1s from cryopreserved monocytes. Two samples of purified allogenic CD 4 cells (1x10⁶/well) were cocultured with cryopreserved TLR agonist stimulated DCs (1x10⁵/well) in comparison to DC1 prepared from cryopreserved monocytes. After 9 days the T cells were harvested and restimulated on plates coated with anti CD3 and anti CD28 antibody. IFNγ levels (produced by the T cells) were analyzed in the supernatant 24 hr later.

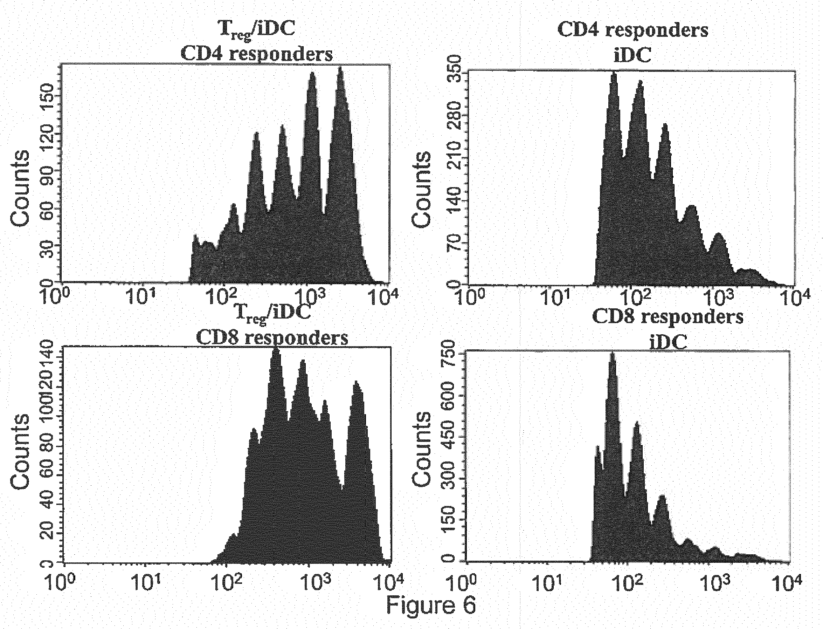

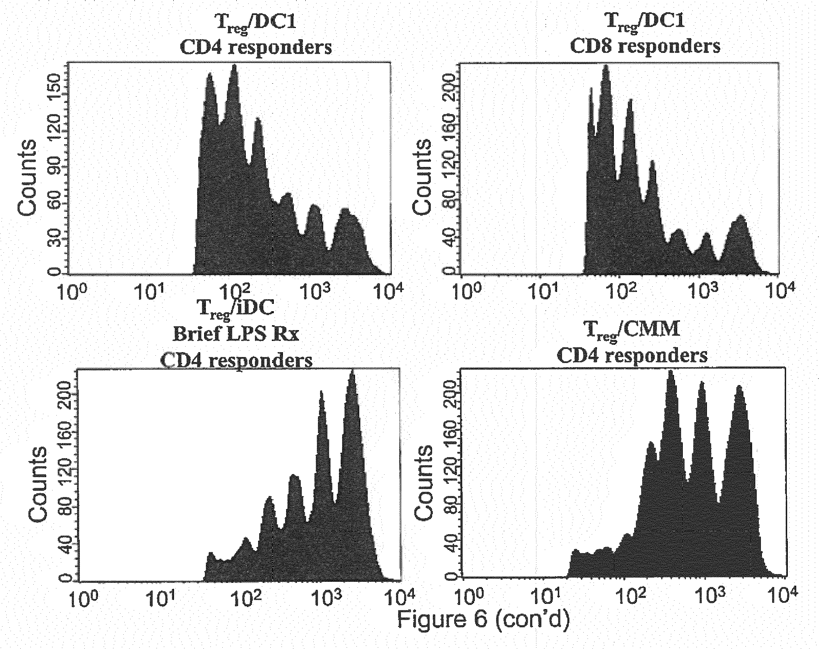

Figure 6 depicts CD4+CD25+ T cells inhibiting responder cell proliferation in the presence of immature but not DC1 dendritic cells. 2.5x10⁵ CFSE-labeled unfractionated responder lymphocytes were co-cultured with 1x10⁵ immature dendritic cells (iDC), dendritic cells matured using IFN-γ/LPS (LPS activated DC), or dendritic cells matured using a conventional cytokine cocktail (CMM) for 5 days. 1.25 x 10⁵ sorted, purified CD4+CD25+ T cells (T_{reg}) were included as noted. Responder cell proliferation is shown for CD4-gated and CD8-gated T cells. Data shown are representative of 10 experiments. Proliferation of CD4-positive responders in the presence of T_{regs} and immature dendritic cells treated briefly with LPS (15 minutes) prior to co-culture is shown as well.

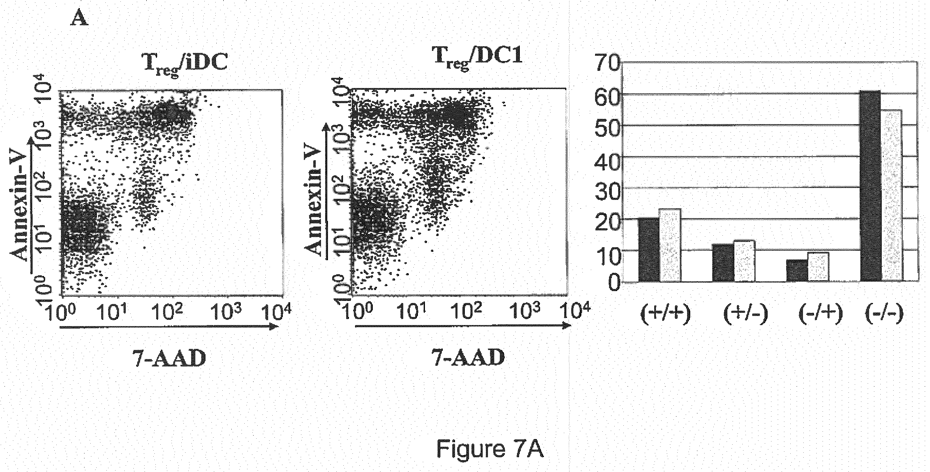

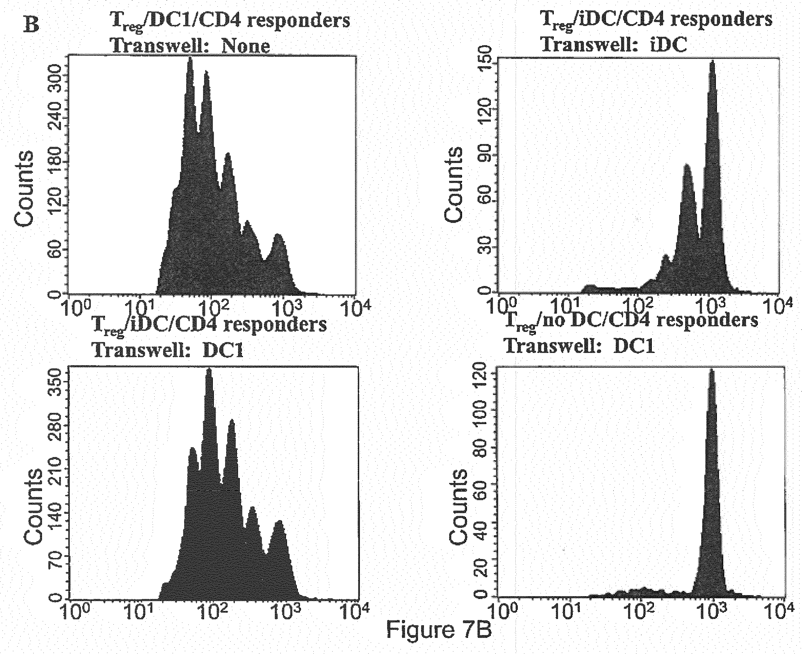

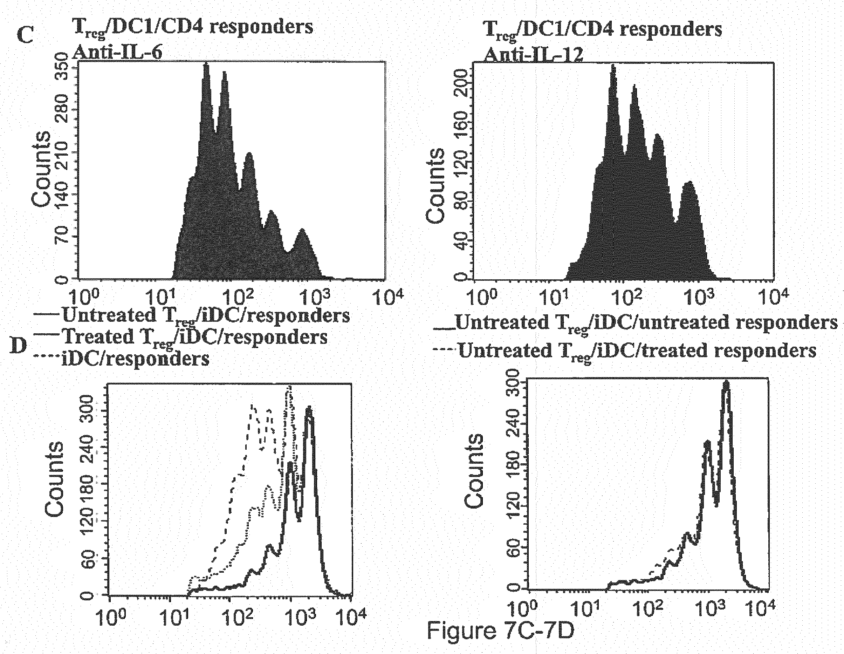

Figure 7, comprising Figures 7A-7D, depicts inhibition of Treg function by DC1 dendritic cells resulting from a soluble factor but is IL-6 and IL-12 independent. Figure 7A depicts 1.25 x 10⁵ sorted, purified T_{regs} co-cultured with 1x10⁵ immature dendritic cells or LPS activated dendritic cells. Expression of the apoptotic markers Annexin-V and 7-AAD 24 hours later is shown. The bar graph summarizes the percent of cells expressing both markers (+/+), Annexin-V only (+/-), 7-AAD only (-/+), or neither marker (-/-). Figure 7B depicts 1.25 x 10⁵ sorted, purified T_{regs} co-cultured with 2.5x10⁵ CFSE-labeled unfractionated responder lymphocytes in the presence of 1x10⁵ immature or LPS activated dendritic cells as noted. In addition, 1x10⁵ immature or LPS activated dendritic cells were added to a semipermeable Transwell® membrane placed in the culture well as noted. Figure 7C depicts 1.25 x 10⁵ sorted, purified T_{regs} co-cultured with 2.5x10⁵ CFSE-labeled unfractionated responder lymphocytes and 1x10⁵ LPS activated dendritic cells in the presence of 5 µg/mL neutralizing anti-IL-6 or anti-IL-12 antibody. The data shown is representative of at least 3 separate experiments in each instance. Figure 7D depicts T_{regs} or CFSE-labeled unfractionated responder lymphocytes cultured in 500µL culture medium and 500 µL of medium taken from LPS activated dendritic cell cultures approximately 10 hours after the addition of LPS (1x10⁶ cells per mL). After 24 hours, these "treated" populations were utilized in co-cultures at the typical ratio (1:2 T_{regs}:responders) as noted. The data is representative of 2 separate experiments.

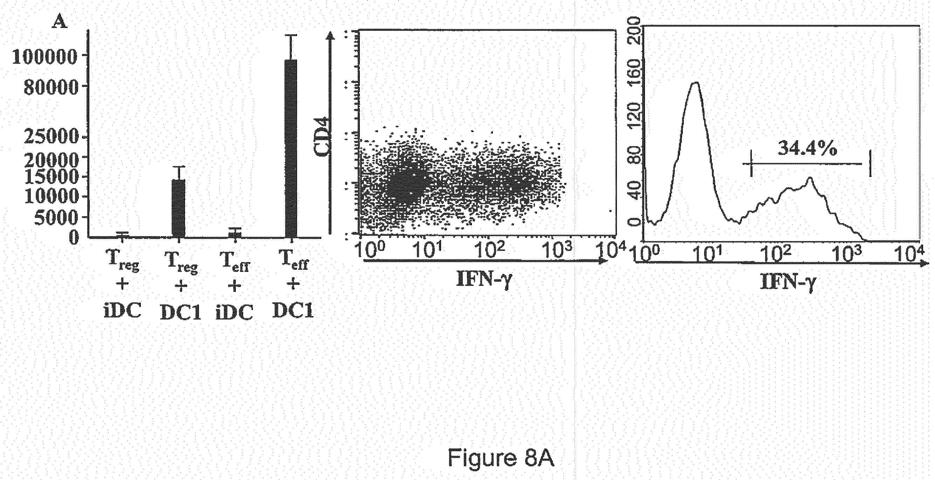

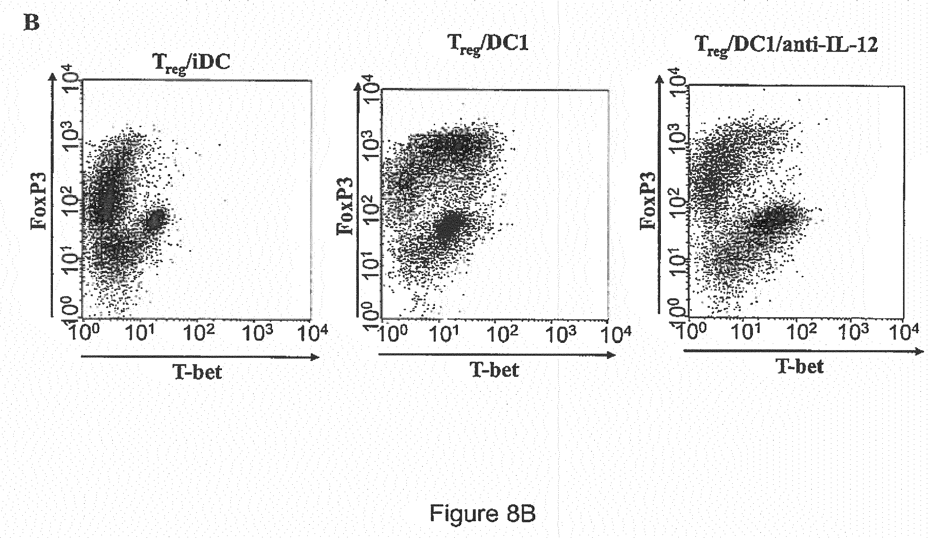

Figure 8, comprising Figures 8A and 8B, depict suppressor CD4+CD25+ T cells secreting effector cytokines in the presence of DC1 dendritic cells. Figure 8A depicts 2.5x10⁵ sorted CD4+CD25+ (T_{reg}) or CD4+CD25- (T_{eff}) T cells combined with 2.0x10⁵ immature or LPS activated dendritic cells. Supernatants were harvested at 5 days and ELISA was used to measure the amount of IFN-γ present in the supernatant. At day 5, some culture samples were permeabilized and intracellular IFN-γ was detected by flow cytometry. 34.4% of CD4 T cells expressed IFN-γ intracellularly (N = 3). Figure 8B depicts 1.25x10⁵ CD4+CD25+ T cells co-cultured with 1x10⁵ immature or LPS activated DC. Neutralizing anti-IL12 antibody (5 µg/mL) was included in some samples. At 48 hours cells were harvested, permeabilized, and intracellular expression of T-bet and FoxP3 was detected by intracellular staining. Data shown are gated on CD4-positive cells. (N = 3)

### DETAILED DESCRIPTION

The present invention relates to the development and cryopreservation of mature, antigen loaded DCs activated by Toll-like receptor agonists to induce clinically effective immune responses, preferably when used earlier in the disease process. The DCs of the present invention have the capacity to condition toward strong Th1 cellular responses, through the production of cytokines and chemokines, and further have the capacity to induce apoptosis of tumor cells. The DC development techniques of the present invention also provide a platform for targeting novel molecules and cancer stem cells that can eliminate cells with high metastatic potential. The present invention also relates to the cryopreservation of these activated DCs in a manner that retains their potency and functionality in presenting antigen as well as their production of various cytokines and chemokines after thawing.

### Definitions

As used herein, each of the following terms has the meaning associated with it in this section.

The articles "a" and "an" are used herein to refer to one or to more than one (i.e. to at least one) of the grammatical object of the article. By way of example, "an element" means one element or more than one element.

The term "about" will be understood by persons of ordinary skill in the art and will vary to some extent on the context in which it is used.

The term "antibody" as used herein, refers to an immunoglobulin molecule, which is able to specifically bind to a specific epitope on an antigen. Antibodies can be intact immunoglobulins derived from natural sources or from recombinant sources and can be immunoactive portions of intact immunoglobulins. Antibodies are typically tetramers of immunoglobulin molecules. The antibodies in the present invention may exist in a variety of forms including, for example, polyclonal antibodies, monoclonal antibodies, Fv, Fab and F(ab)₂, as well as single chain antibodies and humanized antibodies (Harlow et al., 1988; Houston et al., 1988; Bird et al., 1988).

The term "antigen" or "ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to elicit the desired immune response. Moreover, a skilled artisan will understand that an antigen need not be encoded by a "gene" at all. It is readily apparent that an antigen can be generated synthesized or can be derived from a biological sample. Such a biological sample can include, but is not limited to a tissue sample, a tumor sample, a cell or a biological fluid.

"An antigen presenting cell" (APC) is a cell that are capable of activating T cells, and includes, but is not limited to, monocytes/macrophages, B cells and dendritic cells (DCs).

The term "dendritic cell" or "DC" refers to any member of a diverse population of morphologically similar cell types found in lymphoid or non-lymphoid tissues. These cells are characterized by their distinctive morphology and high levels of surface MHC-class II expression. DCs can be isolated from a number of tissue sources. DCs have a high capacity for sensitizing MHC-restricted T cells and are very effective at presenting antigens to T cells in situ. The antigens may be self-antigens that are expressed during T cell development and tolerance, and foreign antigens that are present during normal immune processes.

As used herein, an "activated DC" is a DC that has been exposed to a Toll-like receptor agonist. The activated DC may or may not be loaded with an antigen.

The term "mature DC" as used herein, is defined as a dendritic cell that expresses molecules, including high levels of MHC class II, CD80 (B7.1) and CD86 (B7.2). In contrast, immature dendritic cells express low levels of MHC class II, CD80 (B7.1) and CD86 (B7.2) molecules, yet can still take up an antigen.

"Antigen-loaded APC" or an "antigen-pulsed APC" includes an APC, which has been exposed to an antigen and activated by the antigen. For example, an APC may become Ag-loaded *in vitro*, e.g., during culture in the presence of an antigen. The APC may also be loaded *in vivo* by exposure to an antigen. An "antigen-loaded APC" is traditionally prepared in one of two ways: (1) small peptide fragments, known as antigenic peptides, are "pulsed" directly onto the outside of the APCs; or (2) the APC is incubated with whole proteins or protein particles which are then ingested by the APC. These proteins are digested into small peptide fragments by the APC and are eventually transported to and presented on the APC surface. In addition, the antigen-loaded APC can also be generated by introducing a polynucleotide encoding an antigen into the cell.

The term "autoimmune disease" as used herein is defined as a disorder that results from an autoimmune response. An autoimmune disease is the result of an inappropriate and excessive response to a self-antigen. Examples of autoimmune diseases include but are not limited to, Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, among others.

As used herein, the term "autologous" is meant to refer to any material derived from the same individual to which it is later to be re-introduced into the individual.

The term "cancer" as used herein is defined as disease characterized by the rapid and uncontrolled growth of aberrant cells. Cancer cells can spread locally or through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

The term "cryopreserved" or "cryopreservation" as used herein refers to cells that have been resuspended in a cryomedium and frozen at a temperature of around -70°C or lower.

The term "cryomedium" as used herein refers to any medium mixed with a cell sample in preparation for freezing, such that at least some of cells within the cell sample can be recovered and remain viable after thawing.

"Donor antigen" refers to an antigen expressed by the donor tissue to be transplanted into the recipient.

"Recipient antigen" refers to a target for the immune response to the donor antigen.

As used herein, an "effector cell" refers to a cell which mediates an immune response against an antigen. An example of an effector cell includes, but is not limited to, a T cell and a B cell.

As used herein, "endogenous" refers to any material from or produced inside an organism, cell, tissue or system.

As used herein, the term "exogenous" refers to any material introduced from or produced outside an organism, cell, tissue or system.

The term "epitope" as used herein is defined as a small chemical molecule on an antigen that can elicit an immune response, inducing B and/or T cell responses. An antigen can have one or more epitopes. Most antigens have many epitopes; i.e., they are multivalent. In general, an epitope is roughly five amino acids and/or sugars in size. One skilled in the art understands that generally the overall three-dimensional structure, rather than the specific linear sequence of the molecule, is the main criterion of antigenic specificity and therefore distinguishes one epitope from another.

The term "helper T cell" as used herein is defined as an effector T cell whose primary function is to promote the activation and functions of other B and T lymphocytes and or macrophages. Most helper T cells are CD4 T cells.

As used herein, "immunogen" refers to a substance that is able to stimulate or induce a humoral antibody and/or cell-mediated immune response in a mammal.

The term "immunoglobulin" or "Ig", as used herein is defined as a class of proteins, which function as antibodies. The five members included in this class of proteins are IgA, IgG, IgM, IgD, and IgE. IgA is the primary antibody that is present in body secretions, such as saliva, tears, breast milk, gastrointestinal secretions and mucus secretions of the respiratory and genitourinary tracts. IgG is the most common circulating antibody. IgM is the main immunoglobulin produced in the primary immune response in most mammals. It is the most efficient immunoglobulin in agglutination, complement fixation, and other antibody responses, and is important in defense against bacteria and viruses. IgD is the immunoglobulin that has no known antibody function, but may serve as an antigen receptor. IgE is the immunoglobulin that mediates immediate hypersensitivity by causing release of mediators from mast cells and basophils upon exposure to allergen.

The term "major histocompatibility complex", or "MHC", as used herein is defined as a specific cluster of genes, many of which encode evolutionarily related cell surface proteins involved in antigen presentation, which are among the most important determinants of histocompatibility. Class I MHC, or MHC-I, function mainly in antigen presentation to CD8 T lymphocytes. Class II MHC, or MHC-II, function mainly in antigen presentation to CD4 T lymphocytes.

As used herein, the term "modulate" is meant to refer to any change in biological state, i.e. increasing, decreasing, and the like.

The term "polypeptide" as used herein is defined as a chain of amino acid residues, usually having a defined sequence. As used herein the term polypeptide is mutually inclusive of the terms "peptide" and "protein".

The term "self-antigen" as used herein is defined as an antigen that is expressed by a host cell or tissue. Self-antigens may be tumor antigens, but in certain embodiments, are expressed in both normal and tumor cells. A skilled artisan would readily understand that a self-antigen may be overexpressed in a cell.

As used herein, a "substantially purified" cell is a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some embodiments, the cells are culture *in vitro*. In other embodiments, the cells are not cultured *in vitro*.

The term "T cell" as used herein is defined as a thymus-derived cell that participates in a variety of cell-mediated immune reactions.

The term "B cell" as used herein is defined as a cell derived from the bone marrow and/or spleen, B cells can develop into plasma cells which produce antibodies.

The term "Toll like receptor", or "TLR" as used herein is defined as a class of proteins that play a role in the innate immune system. TLRs are single membrane-spanning, non-catalytic receptors that recognize structurally conserved molecules derived from microbes. TLRs activate immune cell responses upon binding to a ligand.

The term "Toll like receptor agonists", or "TLR agonists" as used herein is defined as a ligand that binds to the TLR to activate immune cell response.

As used herein, a "therapeutically effective amount" is the amount of a therapeutic composition sufficient to provide a beneficial effect to a mammal to which the composition is administered.

The term "vaccine" as used herein is defined as a material used to provoke an immune response after administration of the material to an animal, preferably a mammal, and more preferably a human.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

As contemplated herein, the present invention provides a method for generating and cryopreserving DCs with superior functionality in producing stronger signals to T cells, and thus resulting in a more potent DC-based anti-tumor vaccine. By effectively cryopreserving such cells, samples can be stored and thawed for later use, thereby reducing the need for repeated pheresis and elutriation processes during vaccine production. These methods may also be utilized to directly target molecules involved in carcinogenetic signaling pathways and cancer stem cells (CSCs).

The present invention includes mature, antigen loaded DCs activated by Toll-like receptor agonists that induce clinically effective immune responses, preferably when used earlier in the disease process. The DCs of the present invention produce desirable levels of cytokines and chemokines, and further have the capacity to induce apoptosis of tumor cells.

The present invention further demonstrates that TLR ligands not only activate presenting cells, but also inhibit regulatory cells that function to limit adaptive responses. In certain embodiments, signaling through multiple Toll-like receptors including TLR-2, TLR-4, TLR-8, and TLR-9 has been shown to reverse suppression by immunoregulatory CD4+CD25+Foxp3+ T cells (referred to here as T_{regs}). It is demonstrated herein that TLR-4-activated dendritic cells not only inhibit T_{reg} effects on responder cells but also appear to convert the regulators themselves into IFN-γ producing effectors.

The present invention also relates to the cryopreservation of these activated DCs in a manner that retains their potency and functionality in presenting antigen as well as their production of various cytokines and chemokines after thawing, such that the cryopreserved and subsequently thawed activated DCs are as clinically effective as freshly harvested and activated DCs.

### DC-Based Immunotherapy

DCs are derived from pluripotent monocytes that serve as antigen-presenting cells (APCs). DCs are ubiquitous in peripheral tissues, where they are prepared to capture antigens. Upon antigen capture, DCs process the antigen into small peptides and move towards secondary lymphoid organs. It is within the lymphoid organs that DCs present antigen peptides to naive T cells, thereby initiating a cascade of signals that polarizes T cell differentiation. Upon exposure, DCs present antigen molecules bound to either MHC class I or class II binding peptides and activate CD8⁺ or CD4⁺ T cells, respectively (Steinman, 1991, Annu. Rev. Immunol. 9:271-296; Banchereau et al., 1998, Nature392,245-252; Steinman, et al., 2007, Nature 449:419-426; Ginhoux et al., 2007, J. Exp. Med, 204:3133-3146; Banerjee et al., 2006, Blood 108:2655-2661; Sallusto et al., 1999, J. Exp. Med. 189:611-614; Reid et al., 2000, Curr. Opin. Immunol.12:114-121; Bykovskaia et al., 1999, J. Leukoc. Biol. 66:659-666; Clark et al., 2000, Microbes Infect. 2:257-272).

DCs are responsible for the induction, coordination and regulation of the adaptive immune response and also serve to orchestrate communication between effectors of the innate arm and the adaptive arm of the immune system, These features have made DCs strong candidates for immunotherapy. DCs have a unique capacity to sample the environment through macropinocytosis and receptor-mediated endocytosis (Gerner et al., 2008, J. Immunol. 181:155-164; Stoitzner et al., 2008, Cancer Immunol. Immunother 57:1665-1673; Lanzevecchia A., 1996, Curr. Opin. Immunol.8:348-354; Delamarre et al., 2005, Science,307(5715):1630-1634).

DCs also require maturation signals to enhance their antigen-presenting capacity. DCs upregulate the expression of surface molecules, such as CD80 and CD86 (also known as second signal molecules) by providing additional maturation signals, such as TNF-α, CD40L or calcium signaling agents (Czerniecki et al., 1997,. J. Immunol. 159:3823-3837; Bedrosian et al. 2000, J. Immunother. 23:311-320; Mailliard et al., 2004, Cancer Res.64,5934-5937; Brossart et al., 1998, Blood 92:4238-4247; Jin et al., 2004, Hum. Immunol. 65:93-103). It has been established that a mixture of cytokines, including TNF-α, IL-1β, IL-6 and prostaglandin E2 (PGE2), have the ability to mature DC (Jonuleit, et al., 2000, Arch. Derm. Res. 292:325-332). DCs can also be matured with calcium ionophore prior to being pulsed with antigen.

In addition to pathogen-recognition receptors, such as PKR and MDA-5 (Kalali et al., 2008, J. Immunol. 181:2694-2704; Nallagatla et al., 2008, RNA Biol. 5(3):140-144), DCs also contain a series of receptors, known as Toll-like receptors (TLRs), that are also capable of sensing danger from pathogens. When these TLRs are triggered, a series of activational changes are induced in DCs, which lead to maturation and signaling of T cells (Boullart et al. 2008, Cancer Immunol. Immunother. 57(11):1589-1597; Kaisho et al., 2003, Curr. Mol. Med. 3(4):373-385; Pulendran et al., 2001, Science 293(5528):253-256; Napolitani et al., 2005, Nat. Immunol. 6(8):769-776). DCs can activate and extend the various arms of the cell-mediated response, such as natural killer γ-δ T and α-β T cells and, once activated, DCs retain their immunizing capacity (Steinman, 1991, Annu. Rev. Immunol. 9:271-296; Banchereau et al., 1998, Nature 392:245-252; Reid et al., 2000, Curr. Opin. Immunol. 12:114-121; Bykovskaia et al., 1999, J. Leukoc. Biol.66:659-666; Clark et al., 2000, Microbes Infect. 2:257-272).

### DC-Production of Signal

It is believed that mature DCs are more advantageous in activating T cell-mediated immune responses (Jonuleit et al., 2001, Int. J. Cancer. 93:243-251; Prabakaran et al., 2002, Ann. Surg. Oncol. 9:411-418; Xu et al., 2003, J. Immunol. 171:2251-2261). Mature DCs are capable of generating a greater T cell response as compared with immature DCs, in part because specific cytokines are secreted by mature DCs, which potentiate a stronger and more potent T cell response. For example, mature DCs produce IL-12 upon interaction with CD4 T cells (Koch et al., 1996, J. Exp. Med. 184:741-746; Heifler et al., 1996, Eur. J. Immunol. 26:659-668). DCs that secrete Th1-driving cytokines, such as IL-12, IL-18 and IL-23, are referred to as type-1 polarized DCs, or DC1s (Kalinski, et al., 1999, Immunol. Today 20:561-567; Lanzavecchia et al., 2000, Science 290(5489):92-97),

The cytokines produced by mature DCs have varied effects on T cell response. For example, IL-12, a heterodimeric cytokine, is produced by DCs, and is pivotal in producing IFN-γ-secreting CD4⁺ and CD8⁺ T cells and enhancing antibacterial and anti-tumor responses (Gee et al., 2009, Inflamm. Allergy Drug Targets 8:40-52). IL-12 can also inhibit the growth of primary tumor as well as metastatic tumor cells in ovarian carcinoma (OV-HM) murine models (Tatsumi et al., 2001, Cancer Res. 61:7563-7567). IL-12 can also mediate the generation of high-avidity anti-tumor T cells (Xu et al., 2003, J. Immunol. 171:2251-2261), thereby improving anti-tumor T cell function. DCs also produce chemokines as a fourth signal that leads to the accumulation of T cells and further effects T cell responses (Xiaoet al., 2003, Cytokine 23:126-132).

DCs can secrete other cytokines that further influence T cell activation, For example, DCs can secrete IL-1, IL-6 and IL-23, which activate Th17 cells. Th17 cells are a recently defined subset of proinflammatory T cells that contribute to pathogen clearance and tissue inflammation by means of the production of their signature cytokine, IL-17 (Kikly et al., 2006, Curr. Opin. Immunol. 18:670-675). IL-12 production can lead to a more potent Th1 response, whereas IL-23 production can lead to the maturation of Th17 cells. In fact, IL-12 producing DCs can polarize a predominantly Th1 response in the presence of IL-23, yet by contrast, DCs that produce IL-23 in the absence of IL-12 polarize a strong Th17 response (Roses et al., 2008, J. Immunol. 181:5120-5127; Acosta-Rodriguez et al., 2007, Nat. Immunol. 8:639-646). Thus, because specific DC-secreted cytokines have such a profound impact on T-cell function, the importance of delineating the cytokine profile of mature DCs is a far greater measure of what the potential T-cell effectors may result in. Thus, mature DCs can more effectively be characterized by their dominant cytokine production and subsequent effect of signal on T cells, rather than the more traditional characterization solely by expression of surface molecules.

### Generation of a loaded (pulsed) immune cell

The present invention includes a cell that has been exposed or otherwise "pulsed" with an antigen. For example, an APC, such as a DC, may become Ag-loaded *in vitro*, e.g., by culture *ex vivo* in the presence of an antigen, or *in vivo* by exposure to an antigen.

A person skilled in the art would also readily understand that an APC can be "pulsed" in a manner that exposes the APC to an antigen for a time sufficient to promote presentation of that antigen on the surface of the APC. For example, an APC can be exposed to an antigen in the form of small peptide fragments, known as antigenic peptides, which are "pulsed" directly onto the outside of the APCs (Mehta-Damani *et al*., 1994); or APCs can be incubated with whole proteins or protein particles which are then ingested by the APCs. These whole proteins are digested into small peptide fragments by the APC and eventually carried to and presented on the APC surface (Cohen *et al*., 1994). Antigen in peptide form may be exposed to the cell by standard "pulsing" techniques described herein.

Without wishing to be bound by any particular theory, the antigen in the form of a foreign or an autoantigen is processed by the APC of the invention in order to retain the immunogenic form of the antigen. The immunogenic form of the antigen implies processing of the antigen through fragmentation to produce a form of the antigen that can be recognized by and stimulate immune cells, for example T cells. Preferably, such a foreign or an autoantigen is a protein which is processed into a peptide by the APC. The relevant peptide which is produced by the APC may be extracted and purified for use as an immunogenic composition. Peptides processed by the APC may also be used to induce tolerance to the proteins processed by the APC.

The antigen-loaded APC, otherwise known as a "pulsed APC" of the invention, is produced by exposure of the APC to an antigen either *in vitro* or *in vivo*. In the case where the APC is pulsed *in vitro*, the APC can be plated on a culture dish and exposed to an antigen in a sufficient amount and for a sufficient period of time to allow the antigen to bind to the APC. The amount and time necessary to achieve binding of the antigen to the APC may be determined by using methods known in the art or otherwise disclosed herein. Other methods known to those of skill in the art, for example immunoassays or binding assays, may be used to detect the presence of antigen on the APC following exposure to the antigen.

In a further embodiment of the invention, the APC may be transfected with a vector which allows for the expression of a specific protein by the APC. The protein which is expressed by the APC may then be processed and presented on the cell surface. The transfected APC may then be used as an immunogenic composition to produce an immune response to the protein encoded by the vector.

As discussed elsewhere herein, vectors may be prepared to include a specific polynucleotide which encodes and expresses a protein to which an immunogenic response is desired. Preferably, retroviral vectors are used to infect the cells. More preferably, adenoviral vectors are used to infect the cells.

In another embodiment, a vector may be targeted to an APC by modifying the viral vector to encode a protein or portions thereof that is recognized by a receptor on the APC, whereby occupation of the APC receptor by the vector will initiate endocytosis of the vector, allowing for processing and presentation of the antigen encoded by the nucleic acid of the viral vector. The nucleic acid which is delivered by the virus may be native to the virus, which when expressed on the APC encodes viral proteins which are then processed and presented on the MHC receptor of the APC.

As contemplated herein, various methods can be used for transfecting a polynucleotide into a host cell. The methods include, but are not limited to, calcium phosphate precipitation, lipofection, particle bombardment, microinjection, electroporation, colloidal dispersion systems (i.e. macromolecule complexes, nanocapsules, microspheres, beads, and lipid-based systems including oil-in-water emulsions, micelles, mixed micelles, and liposomes). These methods are understood in the art and are described in published literature so as to enable one skilled in the art to perform these methods.

In another embodiment, a polynucleotide encoding an antigen can be cloned into an expression vector and the vector can be introduced into an APC to otherwise generate a loaded APC. Various types of vectors and methods of introducing nucleic acids into a cell are discussed in the available published literature. For example, the expression vector can be transferred into a host cell by physical, chemical or biological means. See, for example, Sambrook et al. (2001, Molecular Cloning: A Laboratory Manual, Cold Spring Harbor Laboratory, New York), and in Ausubel et al. (1997, Current Protocols in Molecular Biology, John Wiley & Sons, New York). It is readily understood that the introduction of the expression vector comprising a polynucleotide encoding an antigen yields a pulsed cell.

The present invention includes various methods for pulsing APCs including, but not limited to, loading APCs with whole antigen in the form of a protein, cDNA or mRNA. However, the invention should not be construed to be limited to the specific form of the antigen used for pulsing the APC. Rather, the invention encompasses other methods known in the art for generating an antigen loaded APC. Preferably, the APC is transfected with mRNA encoding a defined antigen. mRNA corresponding to a gene product whose sequence is known can be rapidly generated *in vitro* using appropriate primers and reverse transcriptase-polymerase chain reaction (RT-PCR) coupled with transcription reactions. Transfection of an APC with an mRNA provides an advantage over other antigen-loading techniques for generating a pulsed APC. For example, the ability to amplify RNA from a microscopic amount of tissue, i.e. tumor tissue, extends the use of the APC for vaccination to a large number of patients.

For an antigenic composition to be useful as a vaccine, the antigenic composition must induce an immune response to the antigen in a cell, tissue or mammal (e.g., a human). As used herein, an "immunological composition" may comprise an antigen (e.g., a peptide or polypeptide), a nucleic acid encoding an antigen (e.g., an antigen expression vector), or a cell expressing or presenting an antigen or cellular component. In particular embodiments the antigenic composition comprises or encodes all or part of any antigen described herein, or an immunologically functional equivalent thereof. In other embodiments, the antigenic composition is in a mixture that comprises an additional immunostimulatory agent or nucleic acids encoding such an agent. Immunostimulatory agents include but are not limited to an additional antigen, an immunomodulator, an antigen presenting cell or an adjuvant. In other embodiments, one or more of the additional agent(s) is covalently bonded to the antigen or an immunostimulatory agent, in any combination. In certain embodiments, the antigenic composition is conjugated to or comprises an HLA anchor motif amino acids.

A vaccine, as contemplated herein, may vary in its composition of nucleic acid and/or cellular components. In a non-limiting example, a nucleic encoding an antigen might also be formulated with an adjuvant. Of course, it will be understood that various compositions described herein may further comprise additional components. For example, one or more vaccine components may be comprised in a lipid or liposome. In another non-limiting example, a vaccine may comprise one or more adjuvants. A vaccine of the present invention, and its various components, may be prepared and/or administered by any method disclosed herein or as would be known to one of ordinary skill in the art, in light of the present disclosure.

It is understood that an antigenic composition of the present invention may be made by a method that is well known in the art, including but not limited to chemical synthesis by solid phase synthesis and purification away from the other products of the chemical reactions by HPLC, or production by the expression of a nucleic acid sequence (e.g., a DNA sequence) encoding a peptide or polypeptide comprising an antigen of the present invention in an *in vitro* translation system or in a living cell. In addition, an antigenic composition can comprise a cellular component isolated from a biological sample. The antigenic composition isolated and extensively dialyzed to remove one or more undesired small molecular weight molecules and/or lyophilized for more ready formulation into a desired vehicle. It is further understood that additional amino acids, mutations, chemical modification and such like, if any, that are made in a vaccine component will preferably not substantially interfere with the antibody recognition of the epitopic sequence.

A peptide or polypeptide corresponding to one or more antigenic determinants of the present invention should generally be at least five or six amino acid residues in length, and may contain up to about 10, about 15, about 20, about 25, about 30, about 35, about 40, about 45 or about 50 residues or so. A peptide sequence may be synthesized by methods known to those of ordinary skill in the art, such as, for example, peptide synthesis using automated peptide synthesis machines, such as those available from Applied Biosystems, Inc., Foster City, CA (Foster City, CA).

Longer peptides or polypeptides also may be prepared, e.g., by recombinant means. In certain embodiments, a nucleic acid encoding an antigenic composition and/or a component described herein may be used, for example, to produce an antigenic composition *in vitro* or *in vivo* for the various compositions and methods of the present invention. For example, in certain embodiments, a nucleic acid encoding an antigen is comprised in, for example, a vector in a recombinant cell. The nucleic acid may be expressed to produce a peptide or polypeptide comprising an antigenic sequence. The peptide or polypeptide may be secreted from the cell, or comprised as part of or within the cell.

In certain embodiments, an immune response may be promoted by transfecting or inoculating a mammal with a nucleic acid encoding an antigen. One or more cells comprised within a target mammal then expresses the sequences encoded by the nucleic acid after administration of the nucleic acid to the mammal. A vaccine may also be in the form, for example, of a nucleic acid (e.g., a cDNA or an RNA) encoding all or part of the peptide or polypeptide sequence of an antigen. Expression *in vivo* by the nucleic acid may be, for example, by a plasmid type vector, a viral vector, or a viral/plasmid construct vector.

In another embodiment, the nucleic acid comprises a coding region that encodes all or part of the sequences encoding an appropriate antigen, or an immunologically functional equivalent thereof. Of course, the nucleic acid may comprise and/or encode additional sequences, including but not limited to those comprising one or more immunomodulators or adjuvants.

### Antigens

As contemplated herein, the present invention may include use of any antigen suitable for loading into an APC to elicit an immune response. In one embodiment, tumor antigens may be used. Tumor antigens can be divided into two broad categories: *shared* tumor antigens; and *unique* tumor antigens. Shared antigens are expressed by many tumors, while unique tumor antigens can result from mutations induced through physical or chemical carcinogens, and are therefore expressed only by individual tumors. In certain embodiments, shared tumor antigens are loaded into the DCs of the present invention. In other embodiments, unique tumor antigens are loaded into the DCs of the present invention.

In the context of the present invention, "tumor antigen" refer to antigens that are common to specific hyperproliferative disorders. In certain aspects, the hyperproliferative disorder antigens of the present invention are derived from cancers, including but not limited to, primary or metastatic melanoma, thymoma, lymphoma, sarcoma, lung cancer, liver cancer, non-Hodgkin's lymphoma, Hodgkin's lymphoma, leukemia's, uterine cancer, cervical cancer, bladder cancer, kidney cancer and adenocarcinomas such as breast cancer, prostate cancer, ovarian cancer, pancreatic cancer, and the like.

Malignant tumors express a number of proteins that can serve as target antigens for an immune attack. These molecules include, but are not limited to, tissue-specific antigens such as MART-1, tyrosinase and GP 100 in melanoma, and prostatic acid phosphatase (PAP) and prostate-specific antigen (PSA) in prostate cancer. Other target molecules belong to the group of transformation-related molecules, such as the oncogene HER-2/Neu/ErbB-2. Yet another group of target antigens are onco-fetal antigens, such as carcinoembryonic antigen (CEA). In B cell lymphoma, the tumor-specific idiotype immunoglobulin constitutes a truly tumor-specific immunoglobulin antigen that is unique to the individual tumor. B cell differentiation antigens, such as CD19, CD20 and CD37, are other candidates for target antigens in B cell lymphoma. Some of these antigens (CEA, HER-2, CD 19, CD20, idiotype) have been used as targets for passive immunotherapy with monoclonal antibodies with limited success.

The tumor antigen and the antigenic cancer epitopes thereof may be purified and isolated from natural sources such as from primary clinical isolates, cell lines and the like. The cancer peptides and their antigenic epitopes may also be obtained by chemical synthesis or by recombinant DNA techniques known in the arts. Techniques for chemical synthesis are described in Steward et al. (1969); Bodansky et al. (1976); Meienhofer (1983); and Schroder et al. (1965). Furthermore, as described in Renkvist et al. (2001), there are numerous antigens known in the art. Although analogs or artificially modified epitopes are not specifically described, a skilled artisan recognizes how to obtain or generate them by standard means in the art. Other antigens, identified by antibodies and as detected by the Serex technology (see Sahin et al. (1997) and Chen et al. (2000)), are identified in the database of the Ludwig Institute for Cancer Research.

In yet another embodiment, the present invention may include microbial antigens for presentation by the APCs. As contemplated herein, microbial antigens may be viral, bacterial, or fungal in origin. Examples of infectious virus include: Retroviridae (e.g., human immunodeficiency viruses, such as HIV-I (also referred to as HTLV-III, LAV or HTLV-III/LAV, or HIV-III; and other isolates, such as HIV-LP; Picornaviridae (e.g., polio viruses, hepatitis A virus; enteroviruses, human coxsackie viruses, rhinoviruses, echoviruses); Calciviridae (e.g., strains that cause gastroenteritis); Togaviridae (e.g., equine encephalitis viruses, rubella viruses); Flaviridae (e.g., dengue viruses, encephalitis viruses, yellow fever viruses); Coronaviridae (e.g., coronaviruses); Rhabdoviridae (e.g., vesicular stomatitis viruses, rabies viruses); Filoviridae (e.g., ebola viruses); Paramyxoviridae (e.g., parainfluenza viruses, mumps virus, measles virus, respiratory syncytial virus); Orthomyxoviridae (e.g., influenza viruses); Bungaviridae (e.g., Hantaan viruses, bunga viruses, phleboviruses and Nairo viruses); Arena viridae (hemorrhagic fever viruses); Reoviridae (e.g., reoviruses, orbiviruses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae (herpes simplex virus (HSV) 1 and 2, varicella zoster virus, cytomegalovirus (CMV), herpes virus); Poxviridae (variola viruses, vaccinia viruses, pox viruses); and Iridoviridae (e.g., African swine fever virus); and unclassified viruses (e.g., the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e., Hepatitis C); Norwalk and related viruses, and astroviruses).

Examples of infectious bacteria include: Helicobacter pyloris, Borelia burgdorferi, Legionella pneumophilia, Mycobacteria sps (e.g., M. tuberculosis, M. avium, M. intracellulare, M. kansasii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcus faecalis, Streptococcus bovis, Streptococcus (anaerobic sps.), Streptococcus pneumoniae, pathogenic Campylobacter sp., Enterococcus sp., Haemophilus influenzae, Bacillus anthracis, corynebacterium diphtheriae, corynebacterium sp., Erysipelothrix rhusiopathiae, Clostridium perfringens, Clostridium tetani, Enterobacter aerogenes, Klebsiella pneumoniae, Pasturella multocida, Bacteroides sp., Fusobacterium nucleatum, Streptobacillus moniliformis, Treponema Treponema pertenue, Leptospira, and Actinomyces israelli.

Examples of infectious fungi include: Cryptococcus neoformans, Histoplasma capsulatum, Coccidioides immitis, Blastomyces dermatitidis, Chlamydia trachomatis, and Candida albicans. Other infectious organisms (i.e., protists) including: Plasmodium falciparum and Toxoplasma gondii.

### Activation of DCs

While traditional DC-based vaccines (that have previously dominated clinical trials) matured DCs with a cytokine cocktail mixture including combinations of TNF, IL-6, PGE2 and IL-1β, which ultimately stimulated aseptic inflammation, the present invention instead utilizes TLR agonists to mature the DCs and stimulate production of signal.

According to an aspect of the present invention, the stimulation of DCs with a combination of TLR ligands leads to the production of increased amounts of IL-12. Further, activation of DCs with a combination of TLR agonists can yield a more pronounced CD4 and CD8 T-cell response (Warger et al., 2006, Blood 108:544-550). Thus, the DCs of the present invention can secrete Th1 driving cytokines, such as IL-12, by exposure to these ligands that trigger TLRs. For example, the addition of poly(I:C), a TLR3 agonist to IL-1β, TNF-α, and IFN-γ, can generate a potent type-1 polarized DC, characterized by robust levels of IL-12 production (Heifler et al., 1996, Eur. J. Immunol. 26:659-668). In certain embodiments, antigen can be loaded into the DC prior to TLR agonist exposure. In other embodiments, antigen can be loaded into the DC subsequent to TLR agonist exposure.

According to an aspect of the present invention, a novel, integrated approach is used to generate highly effective DCs that produce strong, anti-tumor immune responses via TLR activation. This approach, which may also be called immune conditioning by activated innate (autologous) transfer (ICAIT), utilizes monocyte-derived DCs that are specially activated with biomolecules that simulate bacterial infection, thereby constructing ICAIT-DCs. This unique activation method endows the DCs with qualities not found in DCs that are matured with a cytokine cocktail of TNF, IL-6, PGE2 and IL-1β (the "traditional maturation"), which also simulates aseptic inflammation (Lombardi et al., 2009, J. Immunol. 182:3372-3379).

In one embodiment, the ICAIT-DCs of the present invention can be activated with the combination of the TLR4 agonist, bacterial lipopolysaccharide (LPS), the TLR7/8 agonist, resimiquod (R848) and/or IFN-γ (Amati et al., 2006, Curr. Pharm. Des 12:4247-4254). By activating DCs with a TLR4 agonist and bacterial LPS, ICAIT-DCs are generated that are at least virtually identical (in phenotype) to DC1s generated via traditional maturation methods. These ICAIT-DCs have a high expression of surface molecules, including CD83, CD80, CD86 and HLA-DR. In other embodiments, TLR2 agonists, such as lipotechoic acid (LTA), TLR3 agonists, such as poly(I:C), and/or other TLR4 agonists, such as MPL, may be used. As contemplated herein, any TLR agonist, or combination of TLR agonists, can be used to active DCs, provided such ligands stimulate the production of cytokine and chemokine signals by the activated DCs, Many other TLR agonists are known in the art and can be found in the published literature for use with the present invention.

Even though there are similarities in phenotype between ICAIT-DCs and traditionally matured DCs, the ICAIT-DCs of the present invention display many marked advantages. For example, as depicted in Figure 1, ICAIT-DCs produce greater levels of TNF, as well as high levels of IL-12, CCL3 (MIP-1α) and CCL4 (MIP-1β), CCL5 (RANTES), and CXCL10 (IP-10), as compared to traditionally matured DCs.

Each of these factors can enhance aspects of anti-tumor immunity. For example, CXCL10 (IP-10) chemo-attracts NK cells that enhance tumor rejection (Zing et al., 2005, J. Interferon Cytokine Res. 25:103-112). TNF and IL-12 are antiangiogenic and starve tumors of blood supply (Albini et al., 2009, J. Transl. Med. 7(5)). IL-12 promotes the development and recruitment of IFN-γ-secreting Th1 cells and activates NK cells, In contrast to this, traditionally matured DCs have very poor expression of all of these biomolecules, but instead strongly express CCL17 (TARC), a chemokine associated with allergic reactions and the recruitment of Th2 cells (Xiao et al., 2003, Cytokine 23:126-132). Also depicted in Figure 1 is the ability of ICAIT-DCs to exhibit a unique killer function that the traditional DCs lack, whereby they are capable of lysing breast cancer lines.

Thus, not only do the ICAIT-DCs of the present invention display the production of cytokines known to be effective anti-tumor molecules, the ICAIT-DCs also positively influence the quality of sensitized T cells. As depicted in Figure 2, while both traditionally matured DCs and ICAIT-DCs can successfully sensitize T cells against tumor antigen, only ICAIT-DCs can condition T cells for actual recognition of HER-2-expressing tumors. This suggests that tumors have mechanisms that protect them from recognition by sensitized T cells by means associated with traditional DC activation, but that these mechanisms can be overcome by T cells sensitized and conditioned by the ICAIT-DC. Thus, ICAIT-DCs exemplify unique qualities not possessed by traditional DCs, and that the ICAIT-DC model allows for the conditioning of superior T-cell sensitization, chemotactic attraction of multilineage effectors to tumor deposits, and assists in the direct destruction of cancer cells.

### Use of ICAIT-DCs in Immunotherapy

Although there has been progress in the development of DC-based cancer vaccines, there remains a significant number of challenges that limit the success of current DC-based cancer vaccines. One of the paramount challenges has been to overcome the ability of tumors to evade the immune system. This can be attributed to the fact that tissue-specific tumor-associated antigens can be weakly immunogenic and, thus, evade the host immune response, or the immune response can immunoedit the tumor, thus eliminating antigen-positive cells leaving behind antigen-negative tumor cells. This process of immunoediting is a method whereby the immune response may sculpt a tumor into a more aggressive phenotype. Immunoediting may explain, in part, why targeting only single tissue-specific antigens has resulted in rather limited clinical success. Along with the concept of targeted immunoediting, the idea of epitope spreading has also garnered recent attention as another possible mechanism to enhance the induction of effector T cells.

Most clinical trials using DCs have targeted only tissue-specific tumor proteins/antigens. However, there are numerous molecules that when overexpressed are associated with neoplastic transformation and have yet to be targeted. For example, survivin, a member of the antiapoptotic family, is understood to be a direct downstream target of the signal transduction and activator of transcription (STAT3) pathway (Gritsko et al., 2006, Clin. Cancer Res. 12:11-19). Direct inhibition of STAT3 signaling blocks the expression of the survivin protein and initiates apoptosis of breast cancer cells. It is also believed that induction of *HER-2*/*neu* overexpression upregulates surviving (Siddiqa et al., 2008, BMC Cancer 8:129), This link between a well-recognized tumor antigen, *HER*-*2*/*neu*, and a signaling pathway gene, survivin, affords great potential to develop a novel immunotherapeutic intervention that targets multiple effectors in the pathogenesis of breast cancer. Other immunogenic peptides of the survivin gene have been identified, so the development of anti-survivin, DC-based therapies via the system and methods of the present invention is very plausible, and in combination with established anti-*HER*-*2*/*neu*, DC-based vaccination can have a profound effect on regression, and possibly prevention of, estrogen-independent breast cancers (Reker et al., 2004, Cancer Biol. Ther. 3:180-183).

The protein HER-1/EGFR is yet another example of a novel molecule that can serve as a target for DC-based vaccines developed, according to the system and method of the present invention. Along with survivin, HER-1/EGFR also serves as a novel non-tissue specific target for DC-based cancer vaccines. The overexpression or mutation of HER-1/EGFR has been implicated in the oncogenesis of a variety of malignancies that range from breast carcinoma, colorectal, brain glioma multiforme, pancreatic adenocarcinoma and non-small-cell lung cancer (Hynes et al., 2009, Curr. Opin, Cell Biol. 21:177-184). DCs can be pulsed with HER-1/EGFR and activated as described herein, and thus an anti-HER-1/EGFR T-cell response can be developed.

Mucin 1 (MUC-1) can also serve as a cancer vaccine target. MUC-1 is an epithelial cell glycoprotein that is highly overexpressed and aberrantly glycosylated in many adenocarcinomas, including breast carcinomas, and biliary and pancreatic adenocarcinomas (Vlad et al., 2004, Adv. Immunol. 82:249-293; von Mensdorff-Pouilly et al., 2000, Int. J. Biol. Markers 15,343-356). Overexpression of MUC-1 has been implicated in tumor invasion and metastasis. Given that overexpression of MUC-1 is associated with a number of hematologic and epithelial malignancies, combining anti-MUC-1 DC-based vaccines with vaccines against tissue-specific tumor targets could yield clinically relevant results. Targeting these molecules can have a profound effect on the proliferation of neoplastic cells, and thus halt the progression of disease. Because DC-based vaccines can include proteins such as survivin and HER-1/EGFR, it is possible that disease progression could be clinically affected using a multi-targeted vaccination approach.

As contemplated herein, CSCs are also immunotherapeutic targets for novel DC-based vaccines of the present invention. It is believed that subpopulations of stem cells drive and sustain various neoplasms (Wicha et al., 2006, Cancer Res. 66:1883-1890). The pathways linked to CSC are believed to lack regulation and therefore generate uncontrolled self-renewal of CSCs, which generate tumors that are resistant to traditional therapies (Eyler et al., 2008, J. Clin. Oncol. 26:2839-2845). Current cancer interventions target differentiated tumor cells, but spare populations of CSCs (Eyler et al., 2008, J. Clin. Oncol. 26:2839-2845), yet it is the populations of CSCs that may initiate disease recurrences and/or limit the therapeutic benefit of standard therapies. Therefore, it is imperative to design new strategies based upon a better understanding of the signaling pathways that control aspects of self-renewal and survival in CSCs in order to identify novel therapeutic targets in these cells. Stem cell markers are identified in a number of human malignancies, including hematologic malignancies and tumors of the brain, prostate, breast, pancreas, head and neck, and colon. In addition to the identification of stem cell markers, pathways that regulate self-renewal and cell development such as Wnt, Notch and Hedgehog are also being analyzed in great detail (Kakarala et al., 2008, J. Clin. Oncol. 26:2813-2820; Medina et al., 2009, Clin. Transl. Oncol. 11:199-207; Bolos et al., 2009, Clin. Trans!. Oncol. 11:11-19; Bisson et al., 2009, Cell Res. 19(6):683-697).

The same mechanisms that are being employed in developing particular tumor-targeted DC vaccines can also be utilized to target molecules that are specific to CSCs. It is believed that not all breast cancer cells are equal and that a subset of breast CSCs may be responsible for developing invasion and metastatic disease, Human breast cancers contain a cellular population characterized by the expression of cell-surface markers CD44⁺/CD24low/lin-, which display stem cell properties (Al-Hajj et al., 2003, Proc. Natl Acad, Sci. USA 100:3983-3988). Among the molecules that regulate stem cell populations is *HER-2*/*neu.* There is a correlation between expression of stem cell marker aldehyde dehydrogenase 1 and *HER-2*/*neu* overexpression. For example, in a series of 477 breast carcinomas, *HER-2*/*neu* overexpression in normal human mammary epithelial cells as well as mammary carcinomas correlates with an increase in the proportion of ALDHI-expressing stem cells (Ginestier et al., 2007, Cell Stem Cell. 5:555-567). This correlation between *HER-2*/*neu* expression and stem cells serves as a perfect example of how identifying particular markers of CSCs can facilitate the development of DC-based vaccines using the system and method of the present invention that not only target tumor antigens, but also aim to eliminate self-regulating cells such as stem cells. Targeting molecules that are invariably linked to CSCs can be effective in prevention of cancer in part by reducing clones of pluripotent cells that may serve as the initiators of carcinogenesis. Taking into consideration the present invention with regard to the development of DC-based vaccines, cancer vaccine development can likewise be aimed at targeting molecules that are particular to CSCs. By targeting molecules expressed in CSCs, there is an opportunity to eliminate clones of cells that, perhaps, are responsible for most of the systemic recurrences and for the failure of current anticancer therapies. To accomplish this, molecules are identified that are particular to CSCs, and then the DC-based immunotherapies can be implemented, as contemplated herein, to target molecules that are particular to stem cells.

### Inhibition of Regulatory Cell Function

The present invention further demonstrates that TLR ligands not only activate presenting cells, but also inhibit regulatory cells that function to limit adaptive responses. In certain embodiments, signaling through multiple Toll-like receptors including TLR-2, TLR-4, TLR-8, and TLR-9 reverses suppression by T_{regs}. It is demonstrated herein that TLR-4-activated dendritic cells not only inhibit T_{reg} effects on responder cells but also appear to convert the regulators themselves into IFN-γ producing effectors,

Dendritic cells activated with LPS and IFN-γ but not cytokine-matured DC negated the inhibitory effects of regulatory T cells on responder cell proliferation. This effect is not due to cell death since expression of apoptotic markers in the presence of TLR-activated DC is unchanged. As demonstrated herein, restoration of responder cell proliferation was observed even when TLR-activated dendritic cells were separated from both regulators and responders by a semi-permeable membrane. The effect is by a soluble factor but is independent of both IL-6 and IL-12. Furthermore, this unknown soluble mediator seems to act at least in part on the regulators themselves rather than responder cells. As demonstrated herein, TLR-activated dendritic cells can induce cytokine production and effector function in T regulatory cells. Regulatory T cells produce a substantial amount of IFN-γ in the presence of TLR-activated dendritic cells but not immature or cytokine-matured dendritic cells. IFN-γ production is associated with upregulation of the Th1 transcriptional regulator T-bet, and a significant fraction of IFN-γ -producing regulators co-express T-bet and FoxP3. While the effects of the LPS-activated dendritic cell on responder cell proliferation were IL-12 independent, upregulation of T-bet is inhibited by a neutralizing anti-IL12 antibody. Thus, monocyte derived DC activated with LPS may direct the phenotype of the immune response in part by inhibiting suppressor T cells and recruiting these regulators into Th1 effectors.

### Cryopreservation

As explained previously, the present invention provides not only a system and method for generating superior APCs via the development of ICAIT-DCs, but also provides a system and method for cryopreserving these activated DCs in a way that retains their ability to produce signals critical to T cell function after thawing. As contemplated herein, the present invention includes a variety of cryopreservation techniques and cryomedia, as would be understood by those skilled in the art. For example, in certain embodiments, the cryomedium for cultured cells can include about 5-10% DMSO or glycerol and 10-50% serum, such as human serum, for example. In other embodiments, the cryomedia can be serum-free. In certain embodiments, controlled rate freezing may be used, while other embodiments can include use of insulated containers in which vials of cells mixed with cryomedia are placed in the freezer, such as at temperatures ranging from about -70°C to -80°C. The present invention provides a method to preserve activated ICAIT-DCs in such a manner so as to further facilitate clinical application of such cells, and to reduce the need for extensive and repeated pherisis and elutriation steps. As contemplated herein, cryopreservation techniques may be used for both small-scale and large-scale batches.

When considering the extensive utility for activated DC, the ability to provide a steady supply of cryopreserved activated DC represents a significant advantage that can facilitate various therapeutic uses of such cells. For example, a large-scale culture of activated DC may be cryopreserved in aliquots of the appropriate size, according to the methods of the present invention, such that individual doses of cells can later be used in any particular immunotherapeutic protocol. In certain embodiments, activated DCs can be cryopreserved for 2-24 weeks at temperatures of approximately -70°C or lower. At lower temperatures, such as at about -120°C or lower, activated DCs can be cryopreserved for at least a year or longer.

In one exemplary embodiment, the DCs are suspended in human serum and approximately 10% DMSO (v/v). Alternatively, other serum types, such as fetal calf serum, may be used. The suspended cells can be aliquoted into smaller samples, such as in 1.8 ml vials, and stored at approximately -70°C or lower. In other embodiments, the cryomedium may include about 20% serum and about 10% DMSO, and suspended cells can be stored at about -180°C. Still further embodiments may include medium containing about 55% oxypolygelatine, which is a plasma expander, about 6% hydroxyethylstarch, and about 5% DMSO. Other exemplary cryomediums may include about 12% DMSO and about 25-30% serum.

While the present invention as described herein may include specific concentrations of serum, it should be understood by those skilled in the art that the exact amount of serum in the cryomedium may vary, and in some embodiments may be entirely absent, but will generally be within the range of about 1% to 30%. Of course, any concentration of serum that results in a cell viability of around 50% and/or a cell recovery of around 50% may be used in any ICAIT-DC composition of the present invention, as well as with any cryopreservation method as described herein. Preferably, cell viability and recovery of at least 60%, more preferably at least about 70%, or even 80% is desired when recovering cryopreserved cells in the selected cryomedium.

Similarly, while the present invention as described herein may include specific concentrations of DMSO, those skilled in the art should recognize that DMSO may be entirely absent in some embodiments, while in other embodiments, concentrations from about 5% to as high as about 20% may be used in the cryomedium and included within the cryopreservation methods described herein. Generally, lower concentrations of DMSO are preferred, such as between about 5% to about 10%. However, any concentration of DMSO that results, after thawing, in cell viability of at least 50% and a cell recovery of at least 50%, and preferably a cell viability and recovery of at least 60%, more preferably about 70%, more preferably about 80% and even more preferably about 90% and higher, may be used.

While the present invention as described herein may include reference to a rate controlled freezing, it should be understood by those skilled in the art that methods of freezing in a rate controlled or non-rate controlled manner can routinely be employed. It should also be understood by those skilled in the art that the various cryopreservation mediums as described herein may either include serum or may be serum free. Examples of serum free media can include XVIVO 10, XVIVO 15, XVIVO 20, StemPro, as well as any commercially available serum free media. When utilizing a serum-free cryomedium, the cryopreservation methods of the present invention are generally free of infectious agents, antibodies and foreign proteins, which may be antigenic, and any other foreign molecule that may typically be found in serum-based cryomedia.

Cryopreservation of antigen loaded, active DCs can occur at any point after activation of the cells with TLR agonist. In one embodiment, the activated DCs are cryopreserved approximately 6-8 hr after exposure to the TLR agonist. Preferably, the time point chosen to cryopreserve the activated cells should be based on the maximization of signal production of the cells, particularly IL-12 production.

### Therapeutic applications

The present invention includes the generation of an antigen loaded, activated APC that produces significant levels of cytokines and chemokines when thawed from cryopreservation, where the antigen loaded and activated APC is used in immunotherapy for a mammal, preferably a human. The response to an antigen presented by an APC may be measured by monitoring the induction of a cytolytic T-cell response, a helper T-cell response, and/or antibody response to the antigen using methods well known in the art.

The present invention includes a method of enhancing the immune response in a mammal comprising the steps of: generating immature DCs from monocytes obtained from a mammal (e.g., a patient); pulsing the immature DCs with a composition comprising an antigenic composition; activating the antigen loaded DCs with at least one TLR agonist; cryopreserving the activated, antigen loaded DCs; thawing the activated, antigen loaded DCs and then administering the activated, antigen loaded DCs to a mammal in need thereof. The composition includes at least an antigen, and may further be a vaccine for *ex vivo* immunization and/or *in vivo* therapy in a mammal. Preferably, the mammal is a human.

*Ex vivo* procedures are well known in the art and are discussed more fully below. Briefly, cells are isolated from a mammal (preferably a human). The cells can be administered to a mammalian recipient to provide a therapeutic benefit, The mammalian recipient may be a human and the cells can be autologous with respect to the recipient. Alternatively, the cells can be allogeneic, syngeneic or xenogeneic with respect to the recipient.

In one embodiment, peripheral blood monocytes are obtained from a patient by combined leukapheresis and elutriation. The monocytes can be cultured in SFM with GM-CSF and IL-4 overnight. The next day, immature DCs can be pulsed with antigen, followed by contacting the DCs with IFN-γ and LPS. The activated DCs can then be suspended in a cryomedium and frozen until ready for use in immunotherapy.

Cryopreserved ICAIT-DCs can be cultured *ex vivo* under conditions effective to generate the percent recovery and percent viability of the cells as compared freshly activated ICAIT-DCs. ICAIT-DCs generated from cryopreserved samples can show similar stability as compared to freshly prepared ICAIT-DCs, Furthermore, comparisons of cryopreserved mature DCs with those of freshly prepared DCs can show virtually identical phenotypes as well as signal secretion profiles. As contemplated herein, ICAIT-DCs can be preserved at both small and large scale for approximately 2 to 24 weeks, in the various cryomediums described herein, at temperatures of approximately -70°C to -80°C, At temperatures below about -120°C, the duration of storage can be extended indefinitely or at least beyond 24 weeks without impacting cell recovery, viability, and functionality of the DCs. For example, in certain embodiments, the activated cells can be preserved for at least one year and still retain their ability to produce signal after thawing. The present invention provides for effective recovery and viability profiles upon thawing the cells, and furthermore the cryopreservation conditions described herein do not affect the ability of ICAIT-DCs to retain their signal profiles as explained herein throughout.

In an exemplary embodiment, cryopreservation may be performed after activation of ICAIT-DCs by re-suspending the cells in pre-cooled human serum and subsequently adding approximately 10% DMSO to the sample. The mixture can then be aliquoted in 1.8 ml vials and frozen at about -80°C in a cryochamber overnight. Vials can then be transferred to liquid nitrogen tanks the following day. After about 2 to 24 weeks of cryopreservation, the frozen ICAIT DCs can be thawed and examined for their recovery and viability. Recovery of such ICAIT DCs can be greater than or equal to about 70% with a viability of greater than or equal to about 70%, In other embodiments, DCs or even monocytes can be cryopreserved prior to cell activation.

Alternatively, the procedure for *ex vivo* expansion of hematopoietic stem and progenitor cells is described in U.S. Pat. No. 5,199,942, which is incorporated herein by reference, can be applied to the cells of the present invention. In addition to the cellular growth factors described in U.S. Pat. No. 5,199,942, other factors such as flt3-L, IL-1, IL-3 and c-kit ligand, can be used for culturing and expansion of the cells.

A variety of cell selection techniques are known for identifying and separating cells from a population of cells. For example, monoclonal antibodies (or other specific cell binding proteins) can be used to bind to a marker protein or surface antigen protein found on the cells. Several such markers or cell surface antigens are known in the art.

### Vaccine Formulations

The present invention further includes vaccine formulations suitable for use in immunotherapy. In certain embodiments, vaccine formulations are used for the prevention and/or treatment of a disease, such as cancer and infectious diseases. In one embodiment, the administration to a patient of a vaccine in accordance with the present invention for the prevention and/or treatment of cancer can take place before or after a surgical procedure to remove the cancer, before or after a chemotherapeutic procedure for the treatment of cancer, and before or after radiation therapy for the treatment of cancer and any combination thereof. In other embodiments, the vaccine formulations may be administrated to a patient in conjunction or combination with another composition or pharmaceutical product. It should be appreciated that the present invention can also be used to prevent cancer in individuals without cancer, but who might be at risk of developing cancer.

The administration of a cancer vaccine prepared in accordance with the present invention, is broadly applicable to the prevention or treatment of cancer, determined in part by the selection of antigens forming part of the cancer vaccine. Cancers that can be suitably treated in accordance with the practices of the present invention include, without limitation, cancers of the lung, breast, ovary, cervix, colon, head and neck, pancreas, prostate, stomach, bladder, kidney, bone, liver, esophagus, brain, testicle, uterus and the various leukemia's and lymphomas.

In one embodiment, vaccines in accordance with this invention can be derived from the tumor or cancer cells to be treated. For example, in the treatment of lung cancer, the lung cancer cells would be treated as described hereinabove to produce a lung cancer vaccine. Similarly, breast cancer vaccine, colon cancer vaccine, pancreas cancer vaccine, stomach cancer vaccine, bladder cancer vaccine, kidney cancer vaccine and the like, would be produced and employed as immunotherapeute agents in accordance with the practices for the prevention and/or treatment of the tumor or cancer cell from which the vaccine was produced.

In another embodiment, vaccines in accordance with the present invention could, as stated, also be prepared to treat various infectious diseases which affect mammals, by collecting the relevant antigens shed into a culture medium by the pathogen. As there is heterogenecity in the type of immunogenic and protective antigens expressed by different varieties of organisms causing the same disease, polyvalent vaccines can be prepared by preparing the vaccine from a pool of organisms expressing the different antigens of importance.

In another embodiment of the present invention, the vaccine can be administered by intranodal injection into groin nodes. Alternatively, and depending on the vaccine target, the vaccine can be intradermally or subcutaneously administered to the extremities, arms and legs, of the patients being treated. Although this approach is generally satisfactory for melanoma and other cancers, including the prevention or treatment of infectious diseases, other routes of administration, such as intramuscularly or into the blood stream may also be used.

Additionally, the vaccine can be given together with adjuvants and/or immuno-modulators to boost the activity of the vaccine and the patient's response. Such adjuvants and/or immuno-modulators are understood by those skilled in the art, and are readily described in available published literature.

As contemplated herein, and depending on the type of vaccine being generated, the production of vaccine can, if desired, be scaled up by culturing cells in bioreactors or fermentors or other such vessels or devices suitable for the growing of cells in bulk. In such apparatus, the culture medium would be collected regularly, frequently or continuously to recover therefrom any materials or antigens before such materials or antigens are degraded in the culture medium.

If desired, devices or compositions containing the vaccine or antigens produced and recovered, in accordance with the present invention, and suitable for sustained or intermittent release could be, in effect, implanted in the body or topically applied thereto for a relatively slow or timed release of such materials into the body.

Other steps in vaccine preparation can be individualized to satisfy the requirements of particular vaccines. Such additional steps will be understood by those skilled in the art. For example, certain collected antigenic materials may be concentrated and in some cases treated with detergent and ultracentrifuged to remove transplantation alloantigens.

These methods described herein are by no means all-inclusive, and further methods to suit the specific application will be apparent to the ordinary skilled artisan. Moreover, the effective amount of the compositions can be further approximated through analogy to compounds known to exert the desired effect.

### EXAMPLES

The invention is now described with reference to the following Examples. These Examples are provided for the purpose of illustration only, and the invention is not limited to these Examples, but rather encompasses all variations which are evident as a result of the teachings provided herein.

The experiments disclosed herein were conducted to explore the effects of cryopreservation on the functionality of antigen loaded, activated DCs when thawed for use in immunotherapy. The thawed cells retain the capacity to condition toward strong Th1 cellular responses, through the production of cytokines and chemokines, and further include the capacity to induce apoptosis of tumor cells.

The materials and methods employed in the experiments and examples disclosed herein are now described,

Cryopreservation of ICAIT DC: DCs were harvested by gentle scraping. All medium and the cells were kept at wet ice at all times. Cells were gently washed by centrifugation at about 800RPM for 10 min. 10x10⁶ cells were carefully suspended in human serum and 10% DMSO and transferred to 1.8ml vials. The cells were frozen in an isopropanol bath in a foam insulated Nalgen container at -80 °C. The freezing condition was established by the laboratory as lowering of the temperature by 1° C /min. The cells can also be frozen in biological freezer equipment, which uses N₂ as freezing source. After 18-24 hr the cells were moved quickly to the vapor phase of a liquid N₂ tank. The vapor area of the tank is externally controlled, so that the temperature is never higher than about -135° C.

Thawing of DCs: The cells were transferred to a 37 ° C water bath. When almost thawed the cells were suspended in ice cold DC medium by gently lowering the pipette with the cells into the medium and drop wise release of the cells into the medium. The cells were centrifuged at 800 RPM for 5 min and resuspended. The cells were counted and ready for use.

Preparation of ICAIT DC for cryopreservation: Freshly elutriated myeloid monocytes were cultured in 6 well microplates (12x10⁶ cells/well). Culture medium consisted of Serum Free Medium (SFM Invitrogen Carlsbad CA), The final concentration of added GMCSF was 50ng/ml and of IL4 is 1000 U/ml. Cells were cultured overnight at 37° C in 5% CO₂. In some batches, the cells were pulsed with the adequate peptides after 16-20 hr and cultured for additional 6-8 hr, after which 1000U/ml IFN-γ was added. Dendritic cells were matured with TLR agonist LPS (TLR 4, 10ng/ml) or R848 (TLR8, 1µg/ml). The maturation time was at least about 6hr. After that, the TLR agonist-activated DCs were ready for cryopreservation or immediate use.

The results of the experiments presented herein are now described.

### Example 1: Cryopreserved activated ICAIT-DCs retain signal production at levels similar to freshly generated ICAIT-DCs

First, the viability and recovery of cryopreserved activated DCs was determined. Using trypan blue exclusion, the viability and recovery rate of cryopreserved ICAIT DCs was determined before cryopreservation and immediately after thawing and washing (by centrifugation) of the cryopreserved DCs. As depicted in Figures 3A and 3B, five single cases of two batches of cryopreserved DCs are shown. The viability was comparable between freshly prepared and cryopreserved DC1, and the recovery rate was generally between about 80-90%. Recovery was much better in the cryopreserved samples than from freshly prepared DC1, because of the loss of cells by harvesting of freshly prepared DC1s.

Next, signal production was compared as between fresh ICAIT DCs and cryopreserved ICAIT DCs, as well as with signal production levels from traditional maturation techniques, such as cytokine mediated DC maturation (CMDCs). As depicted in Figure 4A, the continuing production of signal 3 (IL12) was measured by an ELISA Assay. The cryopreserved ICAIT DCs demonstrated similar significant IL12 production levels. Importantly, IL12 production from the cryopreserved ICAIT DCs was notably higher than either cryopreserved CMDCs or fresh CMDCs, demonstrating that cryopreserved ICAIT DCs retain cytokine and chemokine profiles superior to traditionally matured DCs, and consequently more effective cells in eliciting T cell response. As depicted in Figure 4B, IL12 production levels at 2hr and 12 hr after thawing were similar to IL12 production levels seen prior to cryopreservation.

To determine the allosensitization of CD4 T cells, two samples of purified allogenic CD 4 cells (1x10⁶/well) were co-cultured with cryopreserved TLR agonist stimulated DCs (1x10⁵/well), in comparison to DC1s prepared from cryopreserved monocytes. As depicted in Figure 5, after 9 days, the T cells were harvested and restimulated on plates coated with anti CD3 and anti CD28 antibody. IFN-γ levels (produced from the T cells) were analyzed in the supernatant 24 hr later.

### Example 2: CD4+CD25+T cells inhibit responder cell proliferation in the presence of immature but not DC1 dendritic cells

Previously, it was demonstrated that purified CD14+ elutriated monocytes acquire characteristics of activated DC when treated with DC signaling agents (Czerniecki 1997). More recently it was demonstrated that these monocyte-derived tumor antigen-bearing dendritic cells generated using IFN-γ and the TLR-4 agonist LPS (LPS activated DC) promote a targeted immune response in patients with ductal carcinoma in situ (Czerniecki et al., 2007, Cancer Res 67:1842-1852), Prior studies have consistently demonstrated that TLR agonists including LPS are capable of abrogating immunosuppression mediated by CD4+CD25+Foxp3+ T cells (refs). Thus, it is hypothesized that TLR-activated DC may be capable of reversing T_{reg}-mediated immunosuppression.

To test this hypothesis, the experiments were designed to compare the capacity of human CD4+CD25+ T cells to inhibit the proliferation of CD4 and CD8 lymphocytes to TCR stimulation (anti-CD3) in the presence of immature dendritic cells (iDC) versus LPS activated monocyte-derived DC. 1.25x10⁵ sorted CD4+CD25+ T cells were combined with 2.5x10⁵ CFSE-labeled unfractionated lymphocytes (CD4 and CD8 positive) and 1x10⁵ immature dendritic cells. As depicted in Figure 6, the proliferative response of both CD4 and CD8 positive T cells at day 5 was inhibited in the presence versus the absence of T_{regs}. Proliferation of sorted CD4+CD25-T cells was similarly inhibited excluding the possibility that this result was purely an artifact of using unfractionated responders (data not shown). By contrast, inclusion of DC dendritic cells matured using IFN-γ and LPS restored the proliferation of both CD4 and CD8 positive T cells despite the presence of regulators. Responder proliferation in the presence of T_{regs} and LPS activated DC was similar to that in the absence of the regulatory population. That LPS used to mature the monocyte-derived DC contaminated the co-culture leading to this result was excluded by brief pretreatment of iDC with LPS (15 minutes). Suppression was unaffected in this setting, indicating that the DC population needed to be formally matured and contaminating LPS was not responsible for the result. Notably, dendritic cells matured using a conventional cytokine-based maturation cocktail (IL-1, IL-6, TNF-a, PGE2) did not fully restore proliferation of effectors in the presence of regulators. These results demonstrate that monocyte-derived LPS matured DC inhibits T_{reg}-mediated suppression of both CD4 and CD8-positive T cells responding to anti-CD3.

The use of CFSE and more specifically the labeling of effector cells alone is a central aspect of this experimental model. Proliferation assays used to demonstrate suppression in many other studies suffer from their reliance on tritiated thymidine. It has been demonstrated in numerous reports that regulatory T cells are not completely anergic and in fact have significant proliferative potential especially in the context of inflammatory signals (Brinster et al., 2005, J Immunol 175:7332-7340; Klein et al., 2003, Proc Natl Acad Sci U S A 100:8886-8891). Assays based on tritiated thymidine cannot exclude the possibility that proliferative differences noted are due at least in part to the regulatory component. The CFSE-based assay tracks proliferation specifically amongst effector cells and directly compares the proliferation of these effectors in the presence/absence of T_{regs} and/or varying dendritic cell populations. In doing so, it eliminates the possibility that proliferation by CD4+CD25+ regulators promotes misinterpretation of results.

### Example 3: Inhibition of Tree function by DC1 dendritic cells results from a soluble factor but is IL-6 and IL-12 independent

Experiments were designed to characterize the mechanism by which the DC1 population inhibits T_{reg}-mediated suppression. To determine whether DC1 dendritic cells inhibit T_{reg}-mediated suppression by inducing apoptosis in the regulatory T cell complement, sorted CD4+CD25+ T cells were co-cultured with iDC and LPS activated DC and compared the expression of the apoptotic markers Annexin-V and 7-AAD after 24 hours. Day 1 was chosen as the time point with the presumption that proliferative differences noted at day 5 would result from apoptotic events far earlier. After 24 hours of culture, as depicted in Figure 7A(*p* > 0.2 for the Annexin⁺/7-AAD⁺ and Annexin⁻/7AAD⁻ groups), the expression of both Annexin-V and 7-AAD was similar amongst T_{regs} co-cultured with either dendritic cell population. These data suggest that the LPS activated DC population does not significantly alter T_{reg} apoptosis as compared with immature dendritic cells.

Having established that the effect of LPS activated monocyte-derived DC cells on T_{reg}-mediated suppression is not due to apoptosis, to determine whether this effect was cell-contact dependent or mediated by soluble factors, CFSE-labeled effector cells were co-cultured with unlabeled CD4+CD25+ T cells in the presence of immature dendritic cells then added alternate DC populations separated by a semi-permeable Transwell® membrane. When an additional complement of immature dendritic cells was added to the Transwell® membrane, as depicted in Figure 7B, there was no effect on suppression in the presence of iDC. However, when the LPS activated DC population was added to the Transwell® membrane, a break in T_{reg} mediated suppression that was similar to that seen when T_{regs} were co-cultured directly with these DC was observed. To control for the possibility that DC themselves migrated through the Transwell® to break suppression, regulatory T cells were co-cultured with CFSE-labeled effectors in the absence of dendritic cells but added LPS activated DC separated by the membrane. Very little proliferation was noted. These data collectively suggest that the LPS activated DC population can inhibit T_{reg}-mediated suppression in cell contact-independent fashion.

Without wishing to be bond by any particular theory, it is believed the two most likely soluble mediators for the break in suppression are IL-12 and IL-6. The LPS activated DC population is characterized by secretion of a large amount of IL-12 which is principally involved in the development of Th1 immune responses (Xu et al., 2003, J Immunol 171:2251-2261). IL-6 has been shown to be central in the LPS-mediated reversal of T_{reg}-mediated suppression *in vitro* (Pasare et al., 2003, Science 299:1033-1036). Whether neutralization of IL-6 or IL-12 would restore the inhibitory effects of T_{regs} in the presence of LPS activated DC has been tested, it was found as depicted in Figure 7C, the proliferation of effectors in the presence of T_{regs} and IFN-γ/LPS activated DC was minimally affected by inclusion of a neutralizing anti-IL-12 antibody and was essentially unaffected by inclusion of anti-IL-6. Taken together with the Transwell® experiments, these data suggest that a soluble factor other than IL-6 and IL-12 mediates the break in suppression effected by LPS activated DC and does so in an apoptosis-independent manner.

The preceding experiments are unable to discern whether the DC population acts on the regulators themselves or simply releases responder cells from Treg inhibition. Because the DC effect occurs via a soluble factor, to evaluate whether pretreatment of regulators or responders with media taken from LPS activated DC would reverse T_{reg}-mediated suppression, sorted CD4+CD25+ T cells or CFSE-labeled effector cells were cultured in equal parts culture medium and medium transferred from LPS activated DC cultures. These cells were harvested and washed 24 hours later and then utilized in co-cultures. As depicted in Figure 7D, pre-treating responders with LPS activated DC media had no effect on T_{reg}-mediated suppression. Pre-treatment of the regulators prior to co-culture however somewhat restored responder cell proliferation in their presence. Pre-treating T_{regs} with LPS alone (5 ng/mL) did not augment proliferation (data not shown). These findings collectively suggest that the soluble factor may act at least in part on the regulators themselves to break suppression.

A number of recent studies have demonstrated that a variety of Toll-like receptors including TLR-2, TLR-4, TLR-8, and TLR-9 can abrogate T_{reg}-mediated suppression (Urry et al., 2009, J Clin Invest 119:387-398; Pasare et al., 2003, Science 299:1033-1036; Pasare et al., 2003, Science 299:1033-1036; Sutmuller et al., 2006, J Clin Invest 116:485-494; Peng et al., 2005, Science 309:1380-1384; Porrett et al., 2008, J Immunol 181:1692-1699; LaRosa et al., 2007, Immunol Lett 108:183-188; Pasare et al., 2003, Science 299:1033-1036). These authors demonstrated that dendritic cells activated with LPS secrete soluble factor(s) that release effector cells from suppression by CD4+CD25+ T cells. IL-6 was required for this effect but appeared to act synergistically with one or more other cytokines. The results presented herein are compatible with these findings but extend them in a number of respects. First, the experiments were conducted using human cell populations including a dendritic cell currently in use in immunotherapy. It was observed that a similar effect in a human cell population currently in use in tumor immunotherapy translates these findings directly to the clinical venue. Secondly, while these authors added LPS directly to dendritic cells to induce their effect, the experiments showed that dendritic cells activated with LPS then transferred to co-cultures containing T_{regs} and effectors remain capable of abrogating suppression. This finding indicates an enduring effect on the APC and suggests prolonged secretion of a soluble mediator(s) capable of breaking suppression. In this study, that LPS breaks T_{reg}-mediated suppression by acting on the suppressor population or effectors directly is excluded. Rather, it is the LPS-activated dendritic cell that mediates the effects. Third, the experiments demonstrate an effect which is IL-6 and IL-12-independent but appears mediated by a soluble factor. It is plausible that this factor synergizes with IL-6 in the aforementioned study. Lastly, the experiments suggest that LPS-activated dendritic cells may secrete factors that inactivate the regulators themselves rather than simply releasing effectors from T_{reg} control.

### Example 4: Suppressor CD4+CD25+T cells upregulate T-bet, down regulate FoxP3, and secrete effector cytokines in the presence of DC1 dendritic cells

Recent studies in several experimental models have shown that dendritic cells of various phenotypes are capable of converting regulatory T cells into antigen-specific autoimmune effectors (Baban et al., 2009, J Immunol 183:2475-2483, 18; Radhakrishnan et al., 2008, J Immunol 181:3137-3147). Mechanistically, this finding is characterized by down regulation of the transcriptional regulator FoxP3 and can involve upregulation of effector cytokines. Most consistently noted is conversion of T_{regs} into IL-17-producing effector cells that likely mediate Th17 immunity (Baban et al., 2009, J Immunol 183:2475-2483, 18; Radhakrishnan et al., 2008, J Immunol 181:3137-3147; Beriou et al., 2009, Blood 113:4240-4249). To determine whether the break in suppression reflected simple deactivation of regulatory T cells or their conversion into effector cells that further augment the immune response. Given that IFN- γ/LPS activated DC exhibit robust production of IL-12 and are strong inducers of Th1 immunity, it was hypothesized that conversion into effectors would more likely be characterized by IFN-γ than IL-17 production.

To test this hypothesis, T_{regs} and CD4+CD25- effectors were co-cultured at the typical 1.25:1 ratio and measured their cytokine production using ELISA, It was found that both CD4+ CD25+ T_{regs} and CD4+CD25- effector cells co-cultured with immature dendritic cells made essentially no IFN-γ. However, as depicted in Figure 8A, both populations made significant quantities of IFN-γ when co-cultured with LPS activated monocyte-derived DC. To validate that the cytokine measured was produced by the T cells and not the dendritic cell complement, cells were harvested following co-culture and evaluated the intracellular production of IFN-γ. As depicted in Figure 8A, a significant fraction of CD4+ T cells were IFN-γ-positive. By contrast, a much smaller number of CD11c-positive dendritic cells were cytokine-positive (data not shown). The purity of the sorted CD4+CD25+ population is reliably >99%, ruling out the possibility that cytokine production is mediated by contaminating cells (data not shown). However, a fair percentage of the sorted CD4+CD25+ population is FoxP3-negative (approximately 20%; data not shown). It is therefore plausible that the FoxP3+ cells to which suppression is best ascribed are simply deactivated and that cytokine production comes predominantly from this FoxP3-negative cohort.

Studies demonstrating conversion of T_{regs} to effector cells correlate this phenotypic change with down regulation of the transcriptional regulator FoxP3 and upregulation of a variety of cytokines. This LPS activated DC population is known to polarize a Th1 immune response due to its robust production of IL-12 which fosters development of IFN-γ-producing T cells. Differentiation of Th1 cells is programmed through the action of several transcription factors including the T box factor T-bet. It was hypothesized that conversion of T_{regs} to IFN-γ-producing effector cells in this study may be associated with down regulation of the T_{reg}-specific transcription factor FoxP3 and upregulation of T-bet,

To test this premise, CD4+CD25+ regulators were co-cultured with immature or LPS activated DC and soluble CD3 then used intracellular cytokine staining to analyze expression of FoxP3 and T-bet. As depicted in Figure 8B, compared with immature dendritic cells, LPS activated DC induced upregulation of T-bet amongst the CD4+CD25+ T cells. Interestingly, T-bet upregulation was most notable among FoxP3-positive cells. A significant fraction of CD4+CD25+ T cells were noted at day 2 to express both FoxP3 and T-bet. CD4+CD25+ T cells incubated in the presence of immature dendritic cells did not upregulate T-bet, and very few cells were measured as double positive. A slightly earlier time point (day 2) was chosen under the presumption that differences in proliferation and cytokine production noted at day 5 reflect earlier changes in transcription factor expression.

These data show that production of IFN-γ by CD4+CD25+ T cells co-cultured with LPS activated DC is correlated with upregulation of T-bet especially amongst FoxP3-positive cells. These data also render less likely the possibility that cytokines detected by ELISA are generated by expanding FoxP3 negative CD4+CD25+ T cells. Without wishing to be bound by any particular theory, it is believed that the fact that the majority of T-bet-positive cells are also FoxP3-positive suggests that regulatory T cells are converted into cytokine-producing effectors; less likely is that FoxP3 negative cells expand to generate the cytokine detected. That T_{regs} transitioning to effectors at least transiently express multiple transcriptional regulators has been reported in conversion to Th17 cells (Beriou et al., 2009, Blood 113:4240-4249; Sharma et al., 2009, Blood 113:6102-6111).

Because IL-12 is critical to Th1 differentiation and subsequent production of IFN-γ, To determine whether the conversion of regulators to IFN-γ-producing cells noted here was IL-12 dependent. Although it was shown that these type-1 DC negate the effects of T_{regs} on responder cell proliferation in IL-12-independent fashion, it is feasible that different signals govern conversion to effectors. Therefore T_{regs} was co-cultured with LPS activated DC in the presence of a neutralizing anti-IL-12 antibody and evaluated expression of FoxP3 and T-bet. As depicted in Figure 8B, it was found that T-bet upregulation was minimal in the presence of the neutralizing antibody,

These findings suggest a new paradigm for the integration of regulatory T cells into the immune system. T_{regs} are currently viewed as principal mediators of peripheral tolerance that help to moderate the development of inflammatory immune responses and to prevent autoimmunity. How a productive immune response is initiated despite the presence and activity of these cells is not yet certain. The multitude of studies now demonstrating cessation of T cell regulation in the context of inflammation suggests one model-that inflammatory signals encountered in the setting of pathogenic insult deactivate regulators in developing the immune response. Of note, a TLR-activated DC restores proliferation of responder cells despite the presence of T_{regs}. Recent studies illustrating conversion of T_{regs} into antigen-specific Th17 cells extend this model by suggesting T_{regs} can become a part of the productive immune response (Baban et al., 2009, J Immunol 183:2475-2483; Radhakrishnan et al., 2008, J Immunol 181:3137-3147; Beriou et al., 2009, Blood 113:4240-4249; Sharma et al., 2009, Blood 113:6102-6111) The data presented herein indicates that in the presence of the LPS-activated DC population, T_{regs} are recruited into an effector-like compartment that predominantly secrete IFN-γ and thus appear to better approximate Th1 cells. Interestingly, this finding (unlike the effect noted on the proliferative response) was IL-12-dependent, Collectively, these data raise several possibilities regarding how T_{regs} are integrated into the developing immune response. First is that distinct signals direct cessation of T_{reg} activity versus conversion into cytokine-producing effectors. It is plausible that soluble signals present during the initiation of the immune response determine its magnitude in part by deciding whether T_{regs} passively bystand or contribute as cytokine-producing effectors. Second is that T_{regs} may be converted into various subsets (Th1, Th2, Th17) of antigen-specific effectors depending on the type of immune response mandated by the nature of the insult. On a molecular basis, this may occur through the upregulation of lineage-specific transcription factors (e.g. T-bet), and cells may at least transiently express high levels of factors that direct both the regulatory and the effector phenotype. These cells may then act synergistically with similarly differentiated, liberated FoxP3-negative cells to maximize immunity.

These results also carry import regarding the design of tumor immunotherapy vaccines. Regulatory T cells have proven problematic in our attempts to induce tumor immunity since the 1980s. Several recent studies have shown that a variety of vaccination strategies increase the frequency and/or potency of regulatory T cells (Lacelle et al., 2009, Clin Cancer Res 15:6881-6890; Eggermont, 2009, Clin Cancer Res 15:6745-6747; Francois et al., 2009, Cancer Res 69:4335-4345). Perhaps most notably, dendritic cells matured using a conventional cytokine cocktail often used to generate vaccines were found to expand FoxP3^{high} cells that appear to have suppressor function (Banerjee et al., 2006, Blood 108:2655-2661). This may compromise the development and endurance of anti-tumor immunity. In light of these findings, several recent protocols combine the anti-tumor vaccine with agents that deplete T_{regs}; the combination of agents often carries increased benefit relative to either agent alone (Delluc et al., Cancer Immunol Immunother 58:1669-1677; Yamamoto et al., 2009, Oncol Rep 22:337-343; Morse et al., 2008, Blood 112:610-618; Rech et al., 2009, Ann N Y Acad Sci 1174:99-106; Shimizu et al., 1999, J Immunol 163:5211-5218; Turk et al., 2004, J Exp Med 200:771-782; Dannull et al., 2005, J Clin Invest 1 15:3623-3633). Thus, optimizing vaccine efficacy may require subversion of normal regulatory processes. In this regard, the LPS-activated monocyte-derived dendritic cell vaccine is ideal in that it appears to inhibit rather than activate T cell-mediated suppression and may carry the advantage of converting these cells into tumor-reactive effectors. Although there has been no direct comparison of the LPS activated DC vaccine to dendritic cells vaccines matured using conventional protocols, the effect on T_{reg} function demonstrated here carries the potential to DC vaccine efficiency. Of added benefit is that this quality is inherent and may not harbor the toxicities associated with the variety of exogenous T_{reg}-depleting therapies currently being used in combined protocols.

The disclosures of each and every patent, patent application, and publication cited herein are hereby incorporated herein by reference in their entirety.

While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

The invention is also defined by the following embodiments:
1. A method of generating antigen loaded, activated dendritic cells (DC) for use in immunotherapy, comprising:
   loading at least one antigen into a DC;
   activating the DC with at least one TLR agonist;
   cryopreserving the DC; and
   thawing the DC;
   wherein the DC produces an effective amount of at least one cytokine to generate a T cell response.
2. The method of embodiment 1, wherein the antigen is a tumor antigen.
3. The method of embodiment 1, wherein the antigen is a microbial antigen.
4. The method of embodiment 1, wherein the TLR agonist is LPS.
5. The method of embodiment 1, wherein the cryopreserving comprises freezing the DC at a temperature of about -70°C or lower.
6. The method of embodiment 1, wherein the recovery and viability of the DC after thawing is greater than or equal to about 70%.
7. The method of embodiment 1, wherein the recovery and viability of the DC after thawing is greater than or equal to about 80%.
8. The method of embodiment 1, wherein the DC are cryopreserved for at least about one week.
9. The method of embodiment 1, wherein the cytokine is IL12.
10. The method of embodiment 1, wherein the DC exhibits a killer function whereby the DC are capable of lysing targeted cancer cells.
11. A method of eliciting an immune response in a mammal, the method comprising administering a previously cryopreserved composition comprising an antigen loaded, activated DC into the mammal in need thereof, wherein the DC is antigen loaded and activated prior to being cryopreserved.
12. The method of embodiment 11, wherein the antigen is a tumor antigen.
13. The method of embodiment 11, wherein the antigen is a microbial antigen.
14. The method of embodiment 11, wherein the TLR agonist is LPS.
15. The method of embodiment 11, wherein the cryopreserving comprises freezing the DC at a temperature of about -70°C or lower.
16. The method of embodiment 11, wherein the recovery and viability of the DC after thawing is greater than or equal to about 70%.
17. The method of embodiment 11, wherein the recovery and viability of the DC after thawing is greater than or equal to about 80%.
18. The method of embodiment 11, wherein the DC are cryopreserved for at least about one week.
19. The method of embodiment 11, wherein the cytokine is IL12.
20. The method of embodiment 11, wherein the DC exhibits a killer function whereby the DC are capable of lysing targeted cancer cells.
21. A preservable composition for eliciting an immune response in a mammal, the composition comprising:
   a DC loaded with at least one antigen;
   wherein the DC has been activated by exposure to at least one TLR agonist; and
   wherein the DC produces an effective amount of at least one cytokine to generate a T cell response, irrespective of whether or not the composition has been cryopreserved.
22. The composition of embodiment 21, wherein the antigen is a tumor antigen.
23. The composition of embodiment 21, wherein the antigen is a microbial antigen.
24. The composition of embodiment 21, wherein the TLR agonist is LPS.
25. The composition of embodiment 21, wherein the composition has been cryopreserved at a temperature of about -70°C or lower.
26. The composition of embodiment 25, wherein the recovery and viability of the DC after thawing is greater than or equal to about 70%.
27. The composition of embodiment 25, wherein the recovery and viability of the DC after thawing is greater than or equal to about 80%.
28. The composition of embodiment 25, wherein the composition is cryopreserved for at least about one week.
29. The composition of embodiment 21, wherein the cytokine is 1L12.
30. The composition of embodiment 21, wherein the DC exhibits a killer function whereby the DC are capable of lysing targeted cancer cells.

## Claims

1. A method of generating antigen loaded, activated dendritic cells (DCs) for use in immunotherapy, comprising:
culturing peripheral blood monocytes in serum free medium in the presence of GM-CSF and IL-4 overnight to generate immature DCs;
loading at least one antigen into the immature DCs;
activating the immature DCs with a TLR agonist and IFN-γ; and
cryopreserving the activated DCs, wherein upon thawing the activated DCs are viable.

2. The method of claim 1, wherein the activated DCs produce an effective amount of at least one cytokine to generate a T cell response.

3. The method of claim 2, wherein the cytokine is IL-12.

4. The method of any one of claims 1 to 3, wherein the antigen is a tumor antigen or a microbial antigen.

5. The method of any one of claims 1 to 4, wherein the cryopreserving comprises freezing the activated DCs at a temperature of about -70°C or lower.

6. The method of any one of claims 1 to 5, wherein the recovery and viability of the activated DCs after thawing is greater than or equal to about 80%.

7. The method of any one of claims 1 to 6, wherein the activated DCs are cryopreserved approximately 6-8 hr after exposure to the TLR agonist.

8. The method of claim 7, wherein the TLR agonist is LPS.

9. The method of any one of claims 1 to 8, wherein the cryopreserving comprises suspending the activated DCs in about 5-10% DMSO or glycerol.

10. The method of claim 4, wherein the tumor antigen is a breast cancer antigen.

11. A composition for eliciting an immune response in a mammal, the composition comprising activated DCs obtained by any one of the methods of claims 1 to 10.
